(19) **Europäisches Patentamt European Patent Office Office européen des brevets**

(11) **EP 4 163 919 B1**

(12) **EUROPEAN PATENT SPECIFICATION**

(45) Date of publication and mention
of the grant of the patent:
**07.01.2026 Bulletin 2026/02**

(21) Application number: **22175738.8**

(22) Date of filing: **27.05.2022**

(51) International Patent Classification (IPC):
**G16C 20/30** $^{(2019.01)}$   **G06N 3/00** $^{(2023.01)}$

(52) Cooperative Patent Classification (CPC):
**G16C 20/30; G06N 3/042; G06N 3/0442;
G06N 3/0464; G06N 3/048; G06N 3/09;**
G06N 3/084; G16C 20/70

(54) **SYSTEM AND METHOD FOR MOLECULAR PROPERTY PREDICTION USING EDGE-CONDITIONED GRAPH ATTENTION NEURAL NETWORK**

SYSTEM UND VERFAHREN ZUR VORHERSAGE VON MOLEKULAREN EIGENSCHAFTEN UNTER VERWENDUNG EINES NEURONALEN NETZWERKS MIT KANTENBEDINGTER GRAPHENAUFMERKSAMKEIT

SYSTÈME ET PROCÉDÉ DE PRÉDICTION DE PROPRIÉTÉ MOLÉCULAIRE UTILISANT UN RÉSEAU NEURONAL D'ATTENTION AU GRAPHE CONDITIONNÉ PAR LES BORDS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.10.2021 IN 202121046327**

(43) Date of publication of application:
**12.04.2023 Bulletin 2023/15**

(73) Proprietor: **Tata Consultancy Services Limited Maharashtra (IN)**

(72) Inventors:
• **SAKHINANA, Sagar Srinivas**
411013 Pune, Maharashtra (IN)
• **BUDDHIRAJU, Venkata Sudheendra**
411013 Pune, Maharashtra (IN)
• **NISTALA, Sri Harsha**
411013 Pune, Maharashtra (IN)
• **RUNKANA, Venkataramana**
411013 Pune, Maharashtra (IN)

(74) Representative: **Goddar, Heinz J.
Boehmert & Boehmert
Anwaltspartnerschaft mbB
Pettenkoferstrasse 22
80336 München (DE)**

(56) References cited:
• **JUSTIN GILMER ET AL: "Neural Message Passing for Quantum Chemistry", 4 April 2017 (2017-04-04), pages 1 - 14, XP055700744, Retrieved from the Internet <URL:https://arxiv.org/abs/1704.01212>**
• **PETAR VELICKOVIC ET AL: "Graph Attention Networks", ARXIV.ORG, CORNELL UNIVERSITY LIBRARY, 201 OLIN LIBRARY CORNELL UNIVERSITY ITHACA, NY 14853, 30 October 2017 (2017-10-30), XP081319409**
• **JUN CHEN ET AL: "Edge-Featured Graph Attention Network", ARXIV.ORG, CORNELL UNIVERSITY LIBRARY, 201 OLIN LIBRARY CORNELL UNIVERSITY ITHACA, NY 14853, 19 January 2021 (2021-01-19), XP081862774**

## Description

CROSS-REFERENCE TO RELATED APPLICATIONS AND PRIORITY

The present application claims priority to Indian application, Application No. 202121046327, filed in India on October 11, 2021.

TECHNICAL FIELD

The disclosure herein generally relates to the field of molecular property prediction, and, more particularly, to system and method for molecular property prediction using Edge-Conditioned Graph Attention Neural Networks.

BACKGROUND

Machine learning technique, in specific, supervised learning on drug-like potential molecules has remarkable applications for use in more effective drug discovery. It provides substantial prospects in diminishing the computational complexity which is a key desideratum for prognostication of molecular properties and aid in billion price tag cost reduction of developing a potential drug for treatment.

Message Passing Neural Networks (MPNN's) are non-spectral approach of performing convolution on unstructured molecular graphs. It is a graph-based property prediction framework. It leverages a message passing algorithm and a set-pooling aggregation operator to derive a graph-level representation of the complete input low treewidth chemical graphs to assist in inductive learning tasks. The MPNN's however suffer from inherent limitation to effectively encapsulate the characteristics of the molecular graph. Moreover, due to its high computational complexity, MPNN in not viable for real-time property prediction. JUSTIN GILMER ET AL: "Neural Message Passing for Quantum Chemistry", COMPUTER SCIENCE, 4 April 2017 (2017-04-04), pages 1-14 discloses message passing neural networks for molecular property prediction.

PETAR VELICKOVIC ET AL: "Graph Attention Networks", ARXIV.ORG, CORNELL UNIVERSITY LIBRARY, 201 OLIN LIBRARY CORNELL UNIVERSITY ITHACA, NY 14853, 30 October 2017 discloses graph attention networks for molecular property prediction.

JUN CHEN ET AL: "Edge-Featured Graph Attention Network", ARXIV.ORG, CORNELL UNIVERSITY LIBRARY, 201 OLIN LIBRARY CORNELL UNIVERSITY ITHACA, NY 14853, 19 January 2021 discloses edge-featured graph attention networks.

SUMMARY

Embodiments of the present disclosure present technological improvements as solutions to one or more of the above-mentioned technical problems recognized by the inventors in conventional systems. For example, in one embodiment of the invention, a processor-implemented method according to claim 1 for molecular property prediction using edge-conditioned graph attention neural networks is provided. The method includes accessing, via one or more hardware processors, a database comprising a plurality of molecular graphs associated with a plurality of molecules and a plurality of labels indicative of chemical properties of the plurality of the molecular graphs, wherein each molecular graph of the plurality of molecular graphs comprises a plurality of sink nodes, each sink node of the plurality of sink nodes connected to a plurality of source nodes for passing neural messages through a plurality of edges. Further, the method includes computing, via the one or more hardware processors, attention coefficients indicative of influence of the plurality of source nodes on a sink node of the plurality of sink nodes based on (1) a softmax output of a matrix product of a learnable attention vector and a concatenated matrix of a linearly transformed hidden state of the sink node and (2) a matrix product of a linearly transformed edge-information associated with an edge connecting the source node from amongst the plurality of source nodes with the sink node, relative difference of hidden state vectors of the source node and the sink node by a learnable weight matrix. Furthermore, the method includes computing, via the one or more hardware processors a plurality of neural messages sent from the plurality of source nodes to the sink node as a product of the summation of a set of hidden state vectors of the plurality of source node and the linearly transformed edge-information associated with the edge connecting the source node from amongst the plurality of source nodes with the sink node. Moreover, the method includes determining, via the one or more hardware processors, a weighted sum of the plurality of neural messages from the plurality of source nodes perceived by the sink node to obtain an aggregated single-message vector from the plurality of source nodes, the weighted sum determined through the linearly transformed edge-information using the attention coefficients. Also, the method includes transforming, via the one or more hardware processors, the hidden state vector of the sink node to obtain a node level embedding of the molecular graph, wherein transforming the hidden state vector of the sink node comprises computing summation of (1) the linearly transformed hidden state vector of the sink node

**EP 4 163 919 B1**

parameterized by the product of the attention coefficients and the learnable weight parameter, and (2) the aggregated single-message vector from the plurality of source nodes. Moreover, the method includes determining, via the one or more hardware processors, graph level embedding of the molecular graph using a read-out function from the node level embedding of the molecular graph. Also, the method includes computing, via the one or more hardware processors, the one or more molecular properties by feeding the graph level embedding of the molecular graph to a linear layer.

In another aspect of the invention, a system according to claim 6 for molecular property prediction using edge-conditioned graph attention neural networks is provided. The system includes a memory storing instructions; one or more communication interfaces; and one or more hardware processors coupled to the memory via the one or more communication interfaces, wherein the one or more hardware processors are configured by the instructions to access a database comprising a plurality of molecular graphs associated with a plurality of molecules and a plurality of labels indicative of chemical properties of the plurality of the molecular graphs, wherein each molecular graph of the plurality of molecular graphs comprises a plurality of sink nodes, each sink node of the plurality of sink nodes connected to a plurality of source nodes for passing neural messages through a plurality of edges. Further, the one or more hardware processors are configured by the instructions to compute attention coefficients indicative of influence of the plurality of source nodes on a sink node of the plurality of sink nodes based on (1) a softmax output of a matrix product of a learnable attention vector and a concatenated matrix of a linearly transformed hidden state of the sink node and (2) a matrix product of a linearly transformed edge-information associated with an edge connecting the source node from amongst the plurality of source nodes with the sink node, relative difference of hidden state vectors of the source node and the sink node by a learnable weight matrix. Furthermore, the one or more hardware processors are configured by the instructions to compute a plurality of neural messages sent from the plurality of source nodes to the sink node as a product of the summation of a set of hidden state vectors of the plurality of source node and the linearly transformed edge-information associated with the edge connecting the source node from amongst the plurality of source nodes with the sink node. Moreover, the one or more hardware processors are configured by the instructions to determine a weighted sum of the plurality of neural messages from the plurality of source nodes perceived by the sink node to obtain an aggregated single-message vector from the plurality of source nodes, the weighted sum determined through the linearly transformed edge-information using the attention coefficients. Also, the one or more hardware processors are configured by the instructions to transform the hidden state vector of the sink node to obtain a node level embedding of the molecular graph, wherein transforming the hidden state vector of the sink node comprises computing summation of (1) the linearly transformed hidden state vector of the sink node parameterized by the product of the attention coefficients and the learnable weight parameter, and (2) the aggregated single-message vector from the plurality of source nodes. Moreover, the one or more hardware processors are configured by the instructions to determine graph level embedding of the molecular graph using a read-out function from the node level embedding of the molecular graph. Also, the one or more hardware processors are configured by the instructions to compute the one or more molecular properties by feeding the graph level embedding of the molecular graph to a linear layer.

In yet another aspect of the invention, a non-transitory computer readable medium according to claim 11 for a executing a method for molecular property prediction using edge-conditioned graph attention neural networks is provided. The non-transitory computer readable medium includes a plurality of instructions, which when executed, cause the molecular property prediction via the following method. The method steps includes accessing a database comprising a plurality of molecular graphs associated with a plurality of molecules and a plurality of labels indicative of chemical properties of the plurality of the molecular graphs, wherein each molecular graph of the plurality of molecular graphs comprises a plurality of sink nodes, each sink node of the plurality of sink nodes connected to a plurality of source nodes for passing neural messages through a plurality of edges. Further, the method steps include computing, via the one or more hardware processors, attention coefficients indicative of influence of the plurality of source nodes on a sink node of the plurality of sink nodes based on (1) a softmax output of a matrix product of a learnable attention vector and a concatenated matrix of a linearly transformed hidden state of the sink node and (2) a matrix product of a linearly transformed edge-information associated with an edge connecting the source node from amongst the plurality of source nodes with the sink node, relative difference of hidden state vectors of the source node and the sink node by a learnable weight matrix. Furthermore, the method steps include computing, via the one or more hardware processors a plurality of neural messages sent from the plurality of source nodes to the sink node as a product of the summation of a set of hidden state vectors of the plurality of source node and the linearly transformed edge-information associated with the edge connecting the source node from amongst the plurality of source nodes with the sink node. Moreover, the method steps include determining, via the one or more hardware processors, a weighted sum of the plurality of neural messages from the plurality of source nodes perceived by the sink node to obtain an aggregated single-message vector from the plurality of source nodes, the weighted sum determined through the linearly transformed edge-information using the attention coefficients. Also, the method steps include transforming, via the one or more hardware processors, the hidden state vector of the sink node to obtain a node level embedding of the molecular graph, wherein transforming the hidden state vector of the sink node comprises computing summation of (1) the linearly transformed hidden state vector of the sink node parameterized by the product of the attention coefficients and the learnable weight parameter, and (2) the aggregated single-message vector from the plurality of source nodes. Moreover, the method steps include determining graph level embedding of the molecular graph using a read-out function from the node level embedding of the molecular graph. Also, the one or more hardware processors are configured by the instructions to compute the one or more molecular properties by feeding the graph level embedding of the molecular graph to a linear layer.

The invention is defined in the appended claims.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1A illustrates a representation of message passing phase of a conventional message passing neural network (MPNN).
FIG. 1B illustrates a representation of readout phase of a conventional message passing neural network (MPNN).
FIG. 2 illustrates a network implementation of a system for molecular property prediction using edge-conditioned graph attention neural networks according to some embodiments of the present disclosure.
FIGS. 3A and 3B is a flow diagram illustrating a method for molecular property prediction using edge-conditioned graph attention neural networks in accordance with some embodiments of the present disclosure.
FIG. 4 is a block diagram of an exemplary computer system for implementing embodiments consistent with the present disclosure.
FIG. 5 is a flow diagram illustrating XXX in accordance with some embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts.

Convolution Neural Networks (CNNs) have contributed to a broad range of scientific breakthroughs, in the fields as deep residual learning for visual recognition, self-driving cars, optical character recognition engine, acoustic modeling in speech recognition, neural machine translation, etc. In view of the inherent methodology, CNN and its variant structured neural network architectures obtain dimensionality reduction and extract dominant features by performing spatial convolutions on Euclidean domains. The ConvNets input is obliged to, therefore, have a regular and grid-like structure. This restriction hampers the utilization of CNN's to numerous scientific disciplines, where irregular graph structure or manifold data are commonly accessible.

Chemical graph theory treats drug-like organic molecules as non-linear static graphs. Message Passing Neural Networks (MPNN) framework merges several distinct and unique contemporary models that exist in the literature. The MPNNs are an abstraction of a non-spectral approach based Graph Convolution Networks. The MPNNs operate on undirected chemical graphs, $\mathcal{G}^{\mathcal{M}} = (\mathcal{V}^{\mathcal{M}}, \mathcal{E}^{\mathcal{M}})$

Let $\mathcal{N}^{\mathcal{M}}(|\mathcal{V}^{\mathcal{M}}|)$ denote the number of nodes and $E^{\mathcal{M}}(|\mathcal{E}^{\mathcal{M}}|)$ represents the number of edges for a homogenous chemical graph, $\mathcal{G}^{\mathcal{M}}$. $\mathcal{G}^{\mathcal{M}}$ is described by a set of node features, $N^{\mathcal{M}} \in R^{|V^{M}| \times C}, N_i^{\mathcal{M}} \in R^{1 \times C} \forall i \in \mathcal{V}^{\mathcal{M}}$ and edge features $e_{ij}^{\mathcal{M}} \in R^{|E^{M}| \times Z}$, $\forall (i,j) \in \mathcal{E}^{\mathcal{M}}$. Here, i & j $\in \mathcal{V}^{\mathcal{M}}$ refer to the neighboring nodes of the chemical graph and are connected by an arbitrary edge, $(i,j) \in \mathcal{E}^{\mathcal{M}} \leftrightarrow (j,i) \in \mathcal{E}^{\mathcal{M}}$, $\forall j \in \mathcal{N}(i)$. The chemical graph connectivity $\mathcal{G}^{\mathcal{M}} = (\mathcal{V}^{\mathcal{M}}, \mathcal{E}^{\mathcal{M}})$ is given by the adjacency matrix, $\mathcal{G}_{\mathcal{A}}^{\mathcal{M}}$.

Molecules are represented as annotated undirected chemical graphs. The atoms are considered to be the nodes of the chemical graphs. The bonds connecting adjacent atoms in the chemical graphs correspond to edges. The MPNN framework is leveraged in this particular scientific discipline for mathematical modeling of the drug-like organic molecules. It helps to gain insight and assists in the description of the chemical graph's topology into a characteristic representation of the entire chemical graphs to later aid in the molecular property prediction task. Based on the graph's connectivity, an incident edge in between two neighboring atoms in the chemical compound (or nodes in a chemical graph) acts as both arriving and departing edge tagged by the same label (bond type).

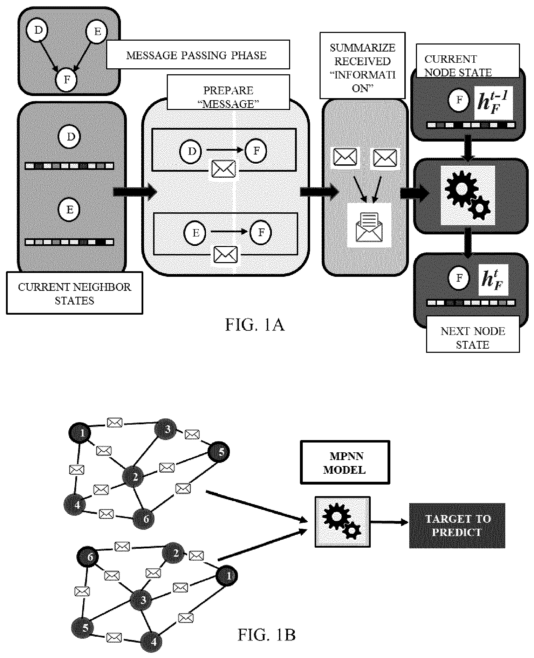

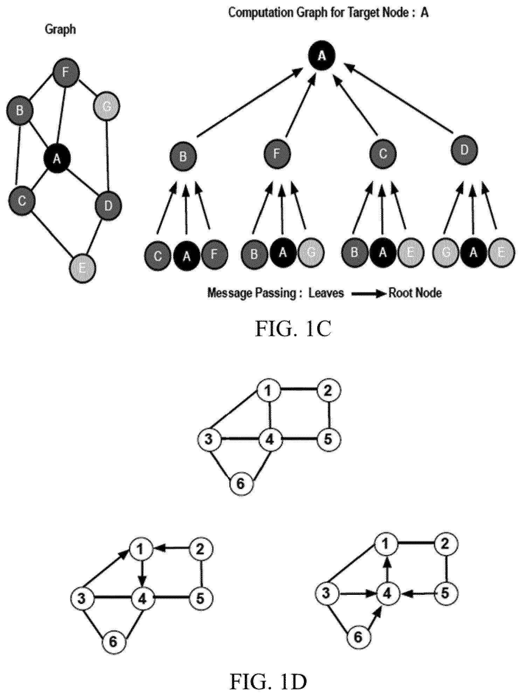

As illustrated in FIGS. 1B, 1C, and 1D, the MPNNs forward pass consists of two phases - a message passing phase (illustrated in FIG. 1C) and a readout phase (illustrated in FIG. 1D). The message passing phase generates neural messages and update node representations by aggregating encoded information of node's embeddings from confined graph neighborhood. A permutation invariant readout phase is leveraged to perform graph pooling. Readout phase function takes an adjacency matrix $\mathcal{G}_A^{\mathcal{M}}$ as input and satisfies the following property, $\mathcal{F}\left(P\mathcal{G}_A^{\mathcal{M}}P^\top\right) = \mathcal{F}\left(\mathcal{G}_A^{\mathcal{M}}\right)$. Here, $P$ is a permutation matrix. The message propagation utilizes the distinct message generating functions acting on the undirected chemical graph topology $M_{in}^f$ and $M_{out}^f$ represents the universal function approximator for the generation of neural messages received through a particular edge type and propagated from the same edge-type between the nodes of the chemical graphs, respectively. Based on the direction of the edge under consideration, that particular transformation function is put into operation on the edge, $e_{ji}^{\mathcal{M}}$. The MPNNs forward pass communicates messages as described by a computational graph for every target node from the bottom (leaf) node to the top (root) node by iterating for T computational time steps. These outgoing messages are evaluated for every edge by transforming the source node's hidden state according to the edge feature vector.

Identical edge-type incident on different node neighbors, which is characterized by distinct edge feature vectors share with the same instance of the message generating neural-network function. Each node in the molecular graph aggregates neural messages (message-pooling operation) from its local T-hop neighbors, as determined from $\mathcal{G}_A^{\mathcal{M}}$ and the received messages are perceived by the target node by performing mathematical computations to update its hidden representation. This message-passing scheme assists in learning effective discriminative hidden representations of the nodes in the chemical graphs, by adopting a Back-propagation through time (BPTT) learning algorithm for training the MPNNs framework when solved for graph-based inductive learning tasks. The MPNNs are synchronous message-passing systems. They update all messages in parallel. In the end, each edge between the vertices in the chemical graph have messages evaluated in both directions from the source to sink and contrariwise. The message passing phase is described by utilizing a message generating function, $M^f$, and node-embedding update neural network function, $V^f$. $M^f$ and $V^f$ might take possession of diverse in specific to be at variance with function settings. During the span of the message passing phase, the node-level embedding $\mathbf{h}_i^t$ of every unique vertex in the molecular graph as given by its computational graph are overhauled and assembled on structural and feature information embedded messages $m_i^{t+1}$, received from its one-hop neighbors as depicted by,

$$m_i^{t+1} = \sum_{j \in N(i)} M^f\left(h_j^t, e_{ji}^{\mathcal{M}}\right) \qquad (1)$$

$$h_i^{t+1} = V^f\left(h_i^t, m_i^{t+1}\right) \qquad (2)$$

Here, $\sum_{j \in N(i)}$ depicts the aggregation of neural-information embedded messages over the local one-hop neighborhood of the node, $i \in \mathcal{V}^{\mathcal{M}}$ in the chemical graph, $\mathcal{G}^{\mathcal{M}}$. Here, $h_i$ is learned with the MPNN model parameters through a representative function of the entire input graph when solved for addressing supervised graph regression tasks such as molecular graph property prediction. The readout phase of the MPNN framework performs graph-pooling through set-pooling approach by determining a discriminative graph embedding for the entire input molecular graph by utilizing a differentiable node-reordering invariant neural network function, $R^f$ according to, $\hat{y} = R^f\left(\{h_i^T | i \in \mathcal{V}^{\mathcal{M}}\left(\mathcal{G}^{\mathcal{M}}\right)\}\right)$. $M^f$, $V^f$, and $R^f$ are differentiable neural network functions and have learnable parameters. Mini-Batching with an MPNN with batch size as a hyper-parameter results in the faster training and augments performance of the graph-based deep learning algorithm. It is viable here in this context as it initiates and propagates neural messages across several molecular graphs with varying numbers of vertices $|\mathcal{V}^{\mathcal{M}}|$ and $|\mathcal{E}^{\mathcal{M}}|$. The feature representation of the vertices in the molecular graph, $\mathcal{G}^{\mathcal{M}}$ is denoted by, *data.x*. *data.edgeindex* describes the edge indices of source and sink vertices of the edge under consideration and vice-versa. *data. edgeattr* represents the static edge attributes. *data.y* is the pre-determined DFT-evaluated properties (ground-truth) for the chemical graphs. The discrepancy between the MPNN model output (estimated) and the true values are measured by the mean-squared error loss function for this graph-based supervised regression task. The Edge-Conditioned Convolution Networks (ECCN) is described by,

$$\mathbf{h}_i^{t+1} = \Lambda \mathbf{h}_i^t + \sum_{j \in N(i)} \mathbf{h}_j^t \cdot \Omega_\Lambda\left(e_{ij}^{\mathcal{M}}\right)$$

Here, $\Omega_\Lambda$ denotes a multilayer perceptron, parameterized by A. The aggregated vector message perceived by the sink node, i is described by $m_i^{t+1} = \sum_{j \in N(i)} \mathbf{h}_j^t \cdot \Omega_\Lambda\left(e_{ij}^{\mathcal{M}}\right)$. The MPNN framework message-generating neural-network function is described by, $M^f\left(h_j^t, e_{ij}^{\mathcal{M}}\right) = \mathrm{h}_j^t \cdot \Omega_\Lambda\left(e_{ij}^{\mathcal{M}}\right)$. The vertex update function is described by, $V^f\left(h_i^t, m_i^{t+1}\right): GRU\left(h_i^t, m_i^{t+1}\right)$. Here, GRU is a known Gated Recurrent Unit. The hidden state of the previous state is given by, $V^f\left(h_i^t, m_i^{t+1}\right): GRU\left(h_i^t, m_i^{t+1}\right)$.

Here, n denotes the total number of nodes in the chemical graphs in a given batch size. $d_m$ & $d_h$ are the characteristic dimension of neural messages and node attributes respectively. Here, the reset gate, $R_t$, $Z_t$ $h_i^t$, $\tilde{h}_i^t$ are evaluated as,

$$\mathrm{R}_t = \sigma(m_i^{t+1}\mathrm{W}_r + h_i^t\mathrm{W}_{rh} + \mathrm{b}_r), \qquad (3)$$

$$\mathrm{Z}_t = \sigma(m_i^{t+1}\mathrm{W}_z + h_i^t\mathrm{W}_{zh} + \mathrm{b}_z) \qquad (4)$$

$$\tilde{h}_i^t = \tanh(m_i^{t+1}\mathrm{W}_{\tilde{h}} + (\mathrm{R}_t \odot h_i^t)\mathrm{W}_{hh} + \mathrm{b}_h) \qquad (5)$$

$$h_i^{t+1} = \mathrm{Z}_t \odot h_i^t + (1 - \mathrm{Z}_t) \odot \tilde{h}_i^t \qquad (6)$$

Here, $\mathrm{W}_r, \mathrm{W}_z, \mathrm{W}_{\tilde{h}} \in \mathbb{R}^{d_m \times d_h}, \mathrm{W}_{rh}, \mathrm{W}_{zh}, \mathrm{W}_{hh} \in \mathbb{R}^{d_h \times d_h}$ are the weight parameters, $\mathrm{b}_r, \mathrm{b}_z, \mathrm{b}_h \in \mathbb{R}^{1 \times d_h}$ are the bias.

The graph-level global pooling neural network is evaluated as

$$\hat{y} = R^f\left(\{h_i^T | i \in \mathcal{V}^{\mathcal{M}}(\mathcal{G}^{\mathcal{M}})\}\right): Set2Set\left(\{h_i^T | i \in \mathcal{V}^{\mathcal{M}}(\mathcal{G}^{\mathcal{M}})\}\right) \quad (7)$$

The MPNN however leads to over-smoothing of learnable embeddings for vertices with higher valency. In addition, the MPNN's suffer from inherent limitation to effectively encapsulate the characteristics of the molecular graph. Moreover, due to its high computational complexity, MPNNs are not viable for real-time molecular property prediction. Various embodiments described herein provides a method and system for molecular property prediction using an edge-conditioned graph attention neural networks. The disclosed system is resilient to noise by learning to adapt to kingpin on the task-relevant fragment of the molecular graphs at varying receptive fields, locality, and depth to augment the discriminative power of node and graph-level embeddings. The disclosed method learns the expressive/discriminative node and graph level embeddings to aid in molecular property prediction with a reduced computational complexity.

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments. It is intended that the following detailed description be considered as exemplary only, with the true scope being indicated by the following claims.

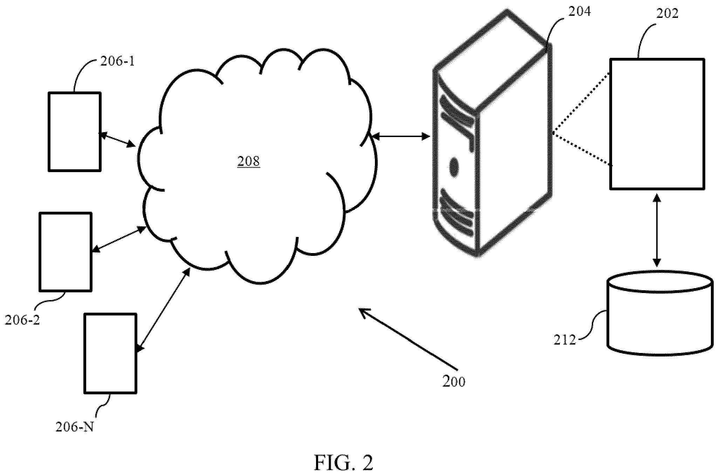

Referring now to the drawings, and more particularly to FIG. 2 through 5, where similar reference characters denote corresponding features consistently throughout the figures, there are shown preferred embodiments and these embodiments are described in the context of the following exemplary system and/or method.

FIG. 2 illustrates a network implementation 200 of a system for molecular property prediction using edge-conditioned graph attention neural networks according to some embodiments of the present disclosure. The disclosed system provides a supervised learning on drug-like potential molecules for use in effective drug discovery. It provides substantial prospects in diminishing the computational complexity which is a key desideratum for prognostication of molecular properties and aid in billion price tag cost reduction of developing a potential drug for treatment.

The proposed system comprises of message-passing, vertex update and graph-pooling modules which augment the graph-level prediction tasks accuracy by taking into account the structure of the graph. The proposed design of message-passing pipeline augments the graph-level prediction tasks accuracy by taking into account the structure of the molecular graph.

Although the present disclosure is explained considering that the system 202 is implemented on a server, it may be understood that the system 202 may also be implemented in a variety of computing systems 204, such as a laptop computer, a desktop computer, a notebook, a workstation, a cloud-based computing environment and the like. It will be understood that the system 202 may be accessed through one or more devices 206-1, 206-2... 206-N, collectively referred to as devices 206 hereinafter, or applications residing on the devices 206. Examples of the devices 206 may include, but are not limited to, a portable computer, a personal digital assistant, a handheld device, a smartphone, a tablet computer, a workstation and the like. The devices 206 are communicatively coupled to the system 202 through a network 208.

In an embodiment, the network 208 may be a wireless or a wired network, or a combination thereof. In an example, the network 208 can be implemented as a computer network, as one of the different types of networks, such as virtual private network (VPN), intranet, local area network (LAN), wide area network (WAN), the internet, and such. The network 206 may either be a dedicated network or a shared network, which represents an association of the different types of networks that use a variety of protocols, for example, Hypertext Transfer Protocol (HTTP), Transmission Control Protocol/Internet Protocol (TCP/IP), and Wireless Application Protocol (WAP), to communicate with each other. Further, the network 208 may include a variety of network devices, including routers, bridges, servers, computing devices, storage devices. The network devices within the network 208 may interact with the system 202 through communication links.

As discussed above, the system 202 may be implemented in a computing device 204, such as a hand-held device, a laptop or other portable computer, a tablet computer, a mobile phone, a PDA, a smartphone, and a desktop computer. The system 202 may also be implemented in a workstation, a mainframe computer, a server, and a network server. In an embodiment, the system 202 may be coupled to a data repository, for example, a repository 212. The repository 212 may store data processed, received, and generated by the system 202. In an alternate embodiment, the system 202 may include the data repository 212.

The network implementation 200 supports various connectivity options such as BLUETOOTH®, USB, ZigBee and other cellular services. The network environment enables connection of devices 206 such as Smartphone with the server 204, and accordingly with the database 212 using any communication link including Internet, WAN, MAN, and so on. In an exemplary embodiment, the system 202 is implemented to operate as a stand-alone device. In another embodiment, the system 202 may be implemented to work as a loosely coupled device to a smart computing environment.

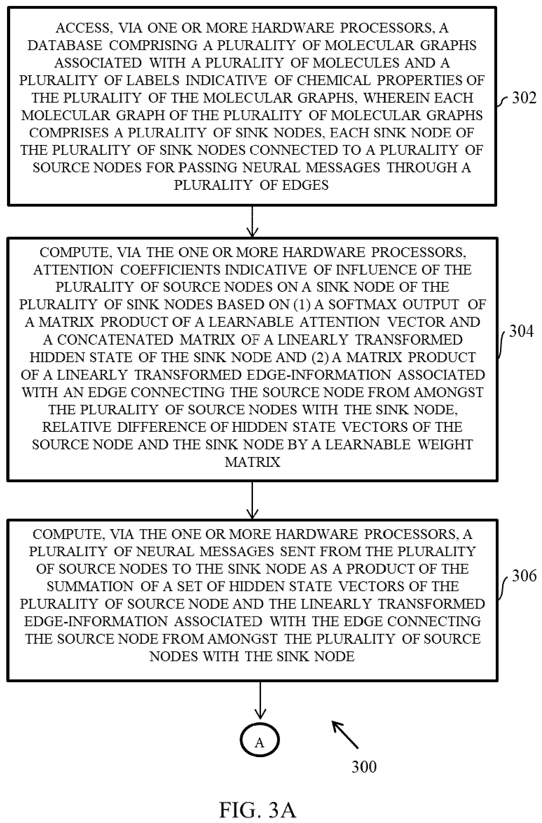

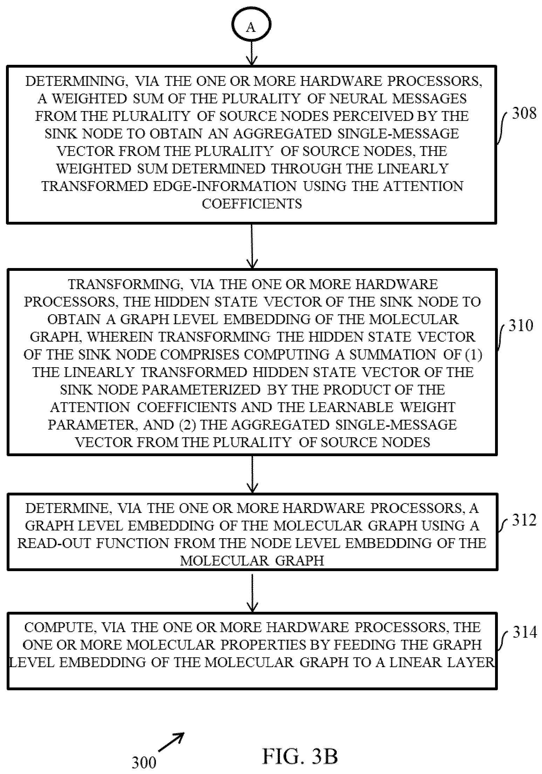

FIGS. 3A and 3B illustrate a flow chart of a method 300 for method for molecular property prediction using edge-conditioned graph attention neural networks, in accordance with an example embodiment of present disclosure. Operations of the flowchart, and combinations of operation in the flowchart, may be implemented by various means, such as hardware, firmware, processor, circuitry and/or other device associated with execution of software including one or more computer program instructions. For example, one or more of the procedures described in various embodiments may be embodied by computer program instructions. In an example embodiment, the computer program instructions, which embody the procedures, described in various embodiments may be stored by at least one memory device of a system and executed by at least one processor in the system. Any such computer program instructions may be loaded onto a computer or other programmable system (for example, hardware) to produce a machine, such that the resulting computer or other programmable system embody means for implementing the operations specified in the flowchart. It will be noted herein that the operations of the method 300 are described with help of system 202. However, the operations of the method 300 can be described and/or practiced by using any other system.

The edge conditioned graph attention networks allow stacking of layers in which each node in the molecular graph aggregate information over their local-graph neighbors attributes, based on molecular graph's connectivity and adaptively adjust to weight nodes of importance. The edge conditioned graph attention network is agnostic to the selection of attention mechanisms and it operates on variable-sized graph structured inputs. It performs self-attention on the nodes of the molecular graph of different sized neighborhood and it also involves a shared attentional mechanism across the nodes of the molecular graphs to compute attention coefficients as depicted by the edge-conditioned graph attentional operator ( $h_i'$ ) in the description below.

At step 302 of method 300, a database comprising a plurality of molecular graphs associated with a plurality of molecules is accessed, via one or more hardware processors. The database further includes a plurality of labels indicative of chemical properties of the plurality of the molecular graphs. Each molecular graph of the plurality of molecular graphs comprises a plurality of sink nodes, each sink node of the plurality of sink nodes connected to a plurality of source nodes for passing neural messages through a plurality of edges. For example, the database may include annotated independent and identically distributed molecular graphs, $D^{\mathcal{M}} = (G_1^{\mathcal{M}}, y_1^{\mathcal{M}}), (G_1^{\mathcal{M}}, y_1^{\mathcal{M}}) \ldots (G_{|D^{\mathcal{M}}|}^{\mathcal{M}}, y_{|D^{\mathcal{M}}|}^{\mathcal{M}})$ .

Here, $y_i^{\mathcal{M}}$ are the associated chemical properties corresponding to the molecular graph, $\mathcal{G}_A^{\mathcal{M}}$. The objective of the graph-based molecular property prediction framework is by operating on the topology of the molecular graphs described by a set of node features, $N^{\mathcal{M}} \in \mathbb{R}^{|V^{\mathcal{M}}| \times c}$ and static edge features, $e_{ij}^{\mathcal{M}} \in \mathbb{R}^{|\mathcal{E}^{\mathcal{M}}| \times z}$ , $\forall (i,j) \in \mathcal{E}^{\mathcal{M}}$ is to learn a novel mapping $f: \mathcal{G}^{\mathcal{M}} \to h_k^{\mathcal{M}}, \forall k \in V^{\mathcal{M}} \to h_{\mathcal{G}}^{\mathcal{M}} \to y_i^{\mathcal{M}}$ that maps molecular graphs structure to the set of

labels. $\mathrm{h}_k^{\mathcal{M}}$ is the learned hidden representation vector of node $k$. Here, $y_i^{\mathcal{M}}$ denotes the target molecular properties.

At 304, the method 300 includes computing, via the one or more hardware processors, attention coefficients indicative of influence of the plurality of source nodes (j) on a sink node (i) of the plurality of sink nodes. The attention coefficients ($\alpha_{i,j}$) are computed based on (1) a softmax output of a matrix product of a learnable attention vector ($a$) and a concatenated matrix ($\Theta_2 h_i$) of a linearly transformed hidden state of the sink node (i) and (2) a matrix product of a linearly transformed edge-information ($e_{j,i}^{\mathcal{M}}$) associated with an edge connecting the source node from amongst the plurality of source nodes with the sink node, relative difference ($h_i$ - $h_j$) of hidden state vectors of the source node and the sink node by a learnable weight matrix ($\Theta_2$), as depicted below:

$$\alpha_{i,j} = \frac{\exp\left(LeakyReLU\left(\mathbf{a}^\top[\boldsymbol{\Theta}_2\mathbf{h}_i \ \| \ e_{j,i}^{\mathcal{M}} \cdot \boldsymbol{\Theta}_2(\mathbf{h}_i - \mathbf{h}_j)]\right)\right)}{\sum_{k \in \mathcal{N}(i) \cup \{i\}} \exp\left(LeakyReLU\left(\mathbf{a}^\top[\boldsymbol{\Theta}_2\mathbf{h}_i \ \| \ e_{k,i}^{\mathcal{M}} \cdot \boldsymbol{\Theta}_2(\mathbf{h}_i - \mathbf{h}_k)]\right)\right)}$$

Here, *LeakyReLU* is a non-linear activation function,

$\mathcal{N}(i)$ represents the plurality of neighboring source nodes perceived by the sink node (*i*).
*a* is a learnable attention vector,
$\Theta_2 h_i$ is concatenated matrix of the linearly transformed hidden state of the sink node (i)

$e_{j,i}^{\mathcal{M}}$ is the edge information of the edge connecting the source node (*j*) with the sink node (*i*),

.$^T$ denotes transposition, and $\|$ represents concatenation.
($h_i$ - $h_j$) is linearly transformed, relative difference of the hidden state of the source node and the sink node,
$\Theta_2$ *is* learnable weight matrix.

The plurality of source nodes sends neural messages to the sink node. The neural messages sent from the source node, *j* to the sink node, *i* is given by the neural net function, $\Gamma_{\Theta'}$, which is parameterized by $\Theta'$. The neural-net function takes as input the summation of hidden state vector ($h_j$) of the source node *j*, and the linearly transformed edge-information given by $\boldsymbol{\Theta}_4 \mathbf{e}_{ji}^{\mathcal{M}}$ to compute attention weighted neural messages. At 306, the method 300 includes computing the plurality of neural messages sent from the plurality of source nodes to the sink node as a product of the summation of a set of hidden state vectors of the plurality of source node and the linearly transformed edge-information $\boldsymbol{\Theta}_4 \mathbf{e}_{ji}^{\mathcal{M}}$ associated with the edge connecting the source node from amongst the plurality of source nodes with the sink node. An attention weighted neural message sent from the source node, *j* to the sink node, *i* is given by the product of the attention coefficients, $\alpha_{i,j}$, the learnable weight matrix, $\Theta_4$, and the message sent from the source node, *j* to the sink node, *i* given $\Gamma_{\Theta'}\left(\mathbf{h}_j + \boldsymbol{\Theta}_4 \mathbf{e}_{ji}^{\mathcal{M}}\right)$.

At 308, the method 300 includes determining a weighted sum of the plurality of neural messages from the plurality of source nodes perceived by the sink node to obtain an aggregated single-message vector from the plurality of source nodes, the weighted sum determined through the linearly transformed edge-information using the attention coefficients. The weighted sum of the plurality of neural messages is given by the equation below:

$$\mathbf{m}_{\mathcal{N}(i)} = \sum_{j \in \mathcal{N}(i)} \alpha_{i,j} \boldsymbol{\Theta}_3 \Gamma_{\Theta'}\left(\mathbf{h}_j + \boldsymbol{\Theta}_4 \mathbf{e}_{ji}^{\mathcal{M}}\right)$$

Here, $\mathbf{m}_{\mathcal{N}(i)}$ represents the weighted sum of the neural messages from the neighboring nodes, $\mathcal{N}(i)$ perceived by the node, *i,* and
$\Theta_4$ is the trainable weight parameter

Herein, the edge information associated with the edge of the molecular graph comprises edge-type characteristics of the edge and a spatial distance between atoms of molecular graph that are represented as nodes.

At 310, the method 300 includes transforming the hidden state vector ($h_i$) of the sink node to obtain a node level embedding ($\mathbf{h}_i'$) of the molecular graph. Herein, transforming the hidden state vector of the sink node comprises computing a summation of (1) the linearly transformed hidden state vector ($h_i$) of the sink node (i) parameterized by the product of the attention coefficients ($\alpha_{i,i}$) and the learnable weight parameter $\Theta_1$, and (2) the aggregated single-message

vector ($\mathbf{m}_{\mathcal{N}(i)}$) from the plurality of source nodes. Mathematically, the node level embedding ($\mathbf{h}_i'$) is denoted as:

$$\mathbf{h}_i' = \alpha_{i,i}\boldsymbol{\Theta}_1\mathbf{h}_i + \sum_{j\in\mathcal{N}(i)} \alpha_{i,j}\boldsymbol{\Theta}_3\left(\Gamma_{\boldsymbol{\Theta}'}\left(\mathbf{h}_j + \boldsymbol{\Theta}_4\mathbf{e}_{ji}^{\mathcal{M}}\right)\right)$$

At 312, the method 300 includes determining, via one or more hardware processors, a graph level embedding of the molecular graph using a read-out function from the node level embedding of the molecular graph. In an embodiment, the read-out function takes as input the node attributes transformed molecular graph and computes the graph-level representation.

In another embodiment, the method at 312 further includes simultaneously operating a spatial-dynamic neighborhood aggregation-based message passing phase on the molecular graph, to exchange the plurality of neural messages resulting in transformation and updating of each node level embedding. Further, a statistical average of the corresponding node level embedding obtained from the edge-conditioned graph attention neural network and the spatial-dynamic neighborhood aggregation-based message passing phase is performed.

In yet another embodiment, the method at 312 further includes simultaneously operating a spatial Identity Mapping graph Convolution Networks based message passing phase on the molecular graph, to exchange the plurality of neural messages resulting in transformation and updating of each node level embedding. Further, a statistical average is performed on the corresponding node level embedding obtained from the edge-conditioned graph attention neural network and the spatial Identity Mapping graph Convolution Networks based message passing phase.

In still another embodiment, the method at 312 further includes simultaneously operating on the molecular graph, a spatial-graph attentional propagation based message passing phase to exchange the plurality of neural messages resulting in transformation and updating of each node level embedding. Further, a statistical average is performed on the corresponding node level embedding obtained from the edge-conditioned graph attention neural network and the spatial-graph attentional propagation based message passing phase.

At 314, the method 300 includes computing, via the one or more hardware processors, the one or more molecular properties by feeding the graph level embedding of the molecular graph to a linear layer.

An example algorithm illustrating steps of the proposed method is presented below:

Edge-Conditioned Graph Attention driven Message-Passing algorithm

**Require**: Molecular Graph $\mathcal{G}^{\mathcal{M}}$

Apply dropout on *data.edgeindex, data.edge - attr*

Apply dropout on *data.x*

\# Message-Passing Phase

**for** $t \le T$ do

Message-Propagation, EC-IMCN

Vertex Update, GRU

**end for**

# Addon Message-Passing Phase

Edge-Conditioned Graph Attention Networks

# Addon Message-Passing Phase

Edge-Conditioned Graph Attentional Propagation

# Read out Operation

Juming Knowledge Networks ([EC-GAP, MPNN,

EI-GAT output])

Graph Structure Incorporated Set2Set Algorithm

**Return** Linear Layer

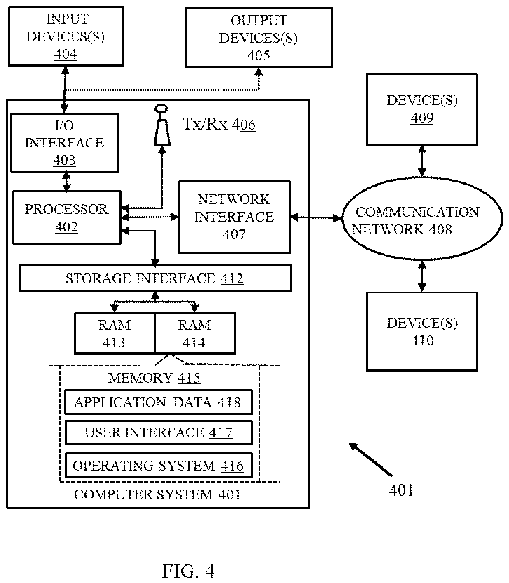

FIG. 4 is a block diagram of an exemplary computer system 401 for implementing embodiments consistent with the present disclosure. The computer system 401 may be implemented in alone or in combination of components of the system 202 (FIG. 2). Variations of computer system 401 may be used for implementing the devices included in this disclosure. Computer system 401 may comprise a central processing unit ("CPU" or "hardware processor" or "processor") 402. The hardware processor 402 may comprise at least one data processor for executing program components for executing user- or system-generated requests. The processor may include specialized processing units such as integrated system (bus) controllers, memory management control units, floating point units, graphics processing units, digital signal processing units, etc. The processor may include a microprocessor, such as AMD Athlon™, Duron™ or Opteron™, ARM's application, embedded or secure processors, IBM PowerPC™, Intel's Core, Itanium™, Xeon™, Celeron™ or other line of processors, etc. The processor 402 may be implemented using mainframe, distributed processor, multi-core, parallel, grid, or other architectures. Some embodiments may utilize embedded technologies like application specific integrated circuits (ASICs), digital signal processors (DSPs), Field Programmable Gate Arrays (FPGAs), etc. The processor 402 may be a multi-core multi-threaded processor.

The processor 402 may be disposed in communication with one or more input/output (I/O) devices via I/O interface 403. The I/O interface 403 may employ communication protocols/methods such as, without limitation, audio, analog, digital, monoaural, RCA, stereo, IEEE-1394, serial bus, universal serial bus (USB), infrared, PS/2, BNC, coaxial, component, composite, digital visual interface (DVI), high-definition multimedia interface (HDMI), RF antennas, S-Video, VGA, IEEE 802.11 a/b/g/n/x, Bluetooth, cellular (e.g., code-division multiple access (CDMA), high-speed packet access (HSPA+), global system for mobile communications (GSM), long-term evolution (LTE), WiMax, or the like), etc.

Using the I/O interface 403, the computer system 401 may communicate with one or more I/O devices. For example, the input device 404 may be an antenna, keyboard, mouse, joystick, (infrared) remote control, camera, card reader, fax machine, dongle, biometric reader, microphone, touch screen, touchpad, trackball, sensor (e.g., accelerometer, light sensor, GPS, gyroscope, proximity sensor, or the like), stylus, scanner, storage device, transceiver, video device/source, visors, etc.

Output device 405 may be a printer, fax machine, video display (e.g., cathode ray tube (CRT), liquid crystal display (LCD), light-emitting diode (LED), plasma, or the like), audio speaker, etc. In some embodiments, a transceiver 406 may be disposed in connection with the processor 402. The transceiver may facilitate various types of wireless transmission or reception. For example, the transceiver may include an antenna operatively connected to a transceiver chip (e.g., Texas Instruments WiLink WL1283, Broadcom BCM4750IUB8, Infineon Technologies X-Gold 618-PMB9800, or the like), providing IEEE 802.11a/b/g/n, Bluetooth, FM, global positioning system (GPS), 2G/3G HSDPA/HSUPA communications, etc.

In some embodiments, the processor 402 may be disposed in communication with a communication network 408 via a network interface 507. The network interface 407 may communicate with the communication network 508. The network interface may employ connection protocols including, without limitation, direct connect, Ethernet (e.g., twisted pair 10/100/1000 Base T), transmission control protocol/internet protocol (TCP/IP), token ring, IEEE 802.11a/b/g/n/x, etc.

The communication network 408 may include, without limitation, a direct interconnection, local area network (LAN), wide area network (WAN), wireless network (e.g., using Wireless Application Protocol), the Internet, etc. Using the network interface 407 and the communication network 408, the computer system 401 may communicate with devices 409 and 410. These devices may include, without limitation, personal computer(s), server(s), fax machines, printers, scanners, various mobile devices such as cellular telephones, smartphones (e.g., Apple iPhone, Blackberry, Android-based phones, etc.), tablet computers, eBook readers (Amazon Kindle, Nook, etc.), laptop computers, notebooks, gaming consoles (Microsoft Xbox, Nintendo DS, Sony PlayStation, etc.), or the like. In some embodiments, the computer system 401 may itself embody one or more of these devices.

In some embodiments, the processor 402 may be disposed in communication with one or more memory devices (e.g., RAM 413, ROM 414, etc.) via a storage interface 412. The storage interface may connect to memory devices including, without limitation, memory drives, removable disc drives, etc., employing connection protocols such as serial advanced technology attachment (SATA), integrated drive electronics (IDE), IEEE-1394, universal serial bus (USB), fiber channel, small computer systems interface (SCSI), etc. The memory drives may further include a drum, magnetic disc drive, magneto-optical drive, optical drive, redundant array of independent discs (RAID), solid-state memory devices, solid-state drives, etc. Variations of memory devices may be used for implementing, for example, any databases utilized in this disclosure.

The memory devices may store a collection of programs or database components, including, without limitation, an operating system 416, a user interface application 417, a user/application data 418 (e.g., any data variables or data records discussed in this disclosure), etc. The operating system 416 may facilitate resource management and operation of the computer system 401. Examples of operating systems include, without limitation, Apple Macintosh OS X, Unix, Unix-like system distributions (e.g., Berkeley Software Distribution (BSD), FreeBSD, NetBSD, OpenBSD, etc.), Linux distributions (e.g., Red Hat, Ubuntu, Kubuntu, etc.), IBM OS/2, Microsoft Windows (XP, Vista/7/8, etc.), Apple iOS, Google Android, Blackberry OS, or the like. The user interface 417 may facilitate display, execution, interaction, manipulation, or operation of program components through textual or graphical facilities. For example, user interfaces may provide computer interaction interface elements on a display system operatively connected to the computer system 401, such as cursors, icons, check boxes, menus, scrollers, windows, widgets, etc. Graphical user interfaces (GUIs) may be employed, including, without limitation, Apple Macintosh operating systems' Aqua, IBM OS/2, Microsoft Windows (e.g., Aero, Metro, etc.), Unix X-Windows, web interface libraries (e.g., ActiveX, Java, Javascript, AJAX, HTML, Adobe Flash, etc.), or the like.

In some embodiments, the computer system 401 may store user/application data 418, such as the data, variables, records, etc. as described in this disclosure. Such databases may be implemented as fault-tolerant, relational, scalable, secure databases such as Oracle or Sybase. Alternatively, such databases may be implemented using standardized data structures, such as an array, hash, linked list, structured text file (e.g., XML), table, or as hand-oriented databases (e.g., using HandStore, Poet, Zope, etc.). Such databases may be consolidated or distributed, sometimes among various computer systems discussed above. It is to be understood that the structure and operation of any computer or database component may be combined, consolidated, or distributed in any working combination.

Additionally, in some embodiments, (the server, messaging and instructions transmitted or received may emanate from hardware, including operating system, and program code (i.e., application code) residing in a cloud implementation. Further, it should be noted that one or more of the systems and methods provided herein may be suitable for cloud-based implementation. For example, in some embodiments, some or all of the data used in the disclosed methods may be sourced from or stored on any cloud computing platform.

Example:

For the purpose of validation, experiments were conducted using QM-9 data set.

The model (as implemented by the disclose system) was trained for graph-level-based regression tasks for the Quantum Chemistry property prediction task. Message passing computational steps, T was constrained to be at 3. The set2set mathematical iterations, M was on par with T. Here, the model trained by leveraging a random selection of datasets for stochastic gradient descent optimization with the Adaptive Moment Estimation optimizer algorithm, with batch size 10. The number of iterations(epochs) is 100 cycles through the full training dataset. The beginning learning rate was chosen as $1e^{-3}$. The learning rate was decayed at 51st epoch by half and maintained it constant in the span of [51; 75] epochs throughout the training and the beginning step size learning rate l decayed to a terminating learning rate $2.5e^{-4}$, using a decay factor by 4 in the range [76; 100] epochs. The QM-9 dataset consists of approximately 134K molecules. The validation set comprises 10000 samples. The test set is composed of 10000 samples and the remaining are for the training set. Here, early stopping is implemented on the validation dataset to prevent the model from over-fitting and for model selection. Finally, the performance of the model was evaluated and the evaluation metric based totally on the test set was published. Feature scaling was performed on the target properties to be predicted. Z-score normalization is leveraged to have distribution mean zero and the expectation of the squared deviation to one for each target property. The gradient descent (aka back-propagation) algorithm was run in weight space by updating the parameters according to the gradients of the loss function, the mean squared error between the predicted model outputs and the predetermined DFT properties. The results are reported in MAE metric in the table below.

Table 1: Performance comparison of the disclosed method with the baseline algorithms on test dataset

| Target | Unit | PPGN | SchNet | PhysNet | MEGNet-s | Comorant | DimeNet | EC-GAT |
|---|---|---|---|---|---|---|---|---|
| Cv | cal/(mol K) | 0.055 | 0.033 | 0.0529 | 0.05 | 0.13 | 0.0286 | 0.0161 |
| G | meV | 36.4 | 14 | 9.40 | 12 | - | 8.98 | 0.0457 |
| H | meV | 36.3 | 14 | 8.42 | 12 | - | 8.11 | 0.0085 |
| HOMO | meV | 40.3 | 41 | 32.9 | 43 | 36 | 27.8 | 0.0043 |
| LUMO | meV | 32.7 | 34 | 24.7 | 44 | 36 | 19.7 | 0.0083 |
| R2 | Bohr2 | 0.592 | 0:073 | 0.765 | 0:302 | 0:673 | 0.331 | 0.0379 |
| U | meV | 36.8 | 14 | 8.15 | 12 | - | 7.89 | 0.0033 |
| U0 | meV | 36.8 | 14 | 8.15 | 12 | - | 8.02 | 0.0034 |
| ZPVE | meV | 3.12 | 1.7 | 1.39 | 1:43 | 1.98 | 1.29 | 0.0000537 |
| alpha | Bohr3 | 0.131 | 0.235 | 0.0615 | 0.081 | 0.092 | 0.0469 | 0.0149 |
| gap | meV | 60.0 | 63 | 42.5 | 66 | 60 | 34.8 | 0.0037 |
| mu | D | 0.047 | 0.033 | 0.0529 | 0:05 | 0.13 | 0:0286 | 0.0157 |

The written description describes the subject matter herein to enable any person skilled in the art to make and use the embodiments. The scope of the subject matter embodiments is defined by the claims.

Also disclosed although not claimed are a program and computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1. A processor implemented method (300) for molecular property prediction using an edge-conditioned graph attention neural networks, comprising:

accessing (302), via one or more hardware processors, a database comprising a plurality of molecular graphs associated with a plurality of molecules and a plurality of labels indicative of chemical properties of the plurality of the molecular graphs, wherein each molecular graph of the plurality of molecular graphs comprises a plurality of sink nodes, each sink node of the plurality of sink nodes connected to a plurality of source nodes for passing neural messages through a plurality of edges, wherein the edge-conditioned graph attention neural networks allow stacking of layers in which each node in the molecular graph aggregate information over their local-graph neighbors attributes based on molecular graph's connectivity and adaptively adjust to weight nodes of importance;

computing (304), via the one or more hardware processors, attention coefficients indicative of influence of the plurality of source nodes on a sink node of the plurality of sink nodes based on (1) a softmax output of a matrix product of a learnable attention vector and a concatenated matrix of a linearly transformed hidden state of the sink node, and (2) a matrix product of a linearly transformed edge-information associated with an edge connecting the source node from amongst the plurality of source nodes with the sink node, relative difference of hidden state vectors of the source node and the sink node by a learnable weight matrix, wherein the edge-conditioned graph attention neural networks performs self-attention on the nodes of the molecular graph of different sized neighborhood and also involve a shared attentional mechanism across the nodes of the molecular graphs to compute attention coefficients, wherein edge-conditioned graph attention neural networks is agnostic to selection of attention mechanism and operates on variable-sized graph structured inputs;

computing (306), via the one or more hardware processors, a plurality of neural messages sent from the plurality of source nodes to the sink node as a product of summation of a set of hidden state vectors of the plurality of source node and the linearly transformed edge-information associated with the edge connecting the source node from amongst the plurality of source nodes with the sink node;

determining (308), via the one or more hardware processors, a weighted sum of the plurality of neural messages from the plurality of source nodes perceived by the sink node to obtain an aggregated single-message vector from the plurality of source nodes, the weighted sum determined through the linearly transformed edge-information using the attention coefficients;

transforming (310), via the one or more hardware processors, the hidden state vector of the sink node to obtain a node level embedding of the molecular graph, wherein transforming the hidden state vector of the sink node comprises computing a summation of (1) the linearly transformed hidden state vector of the sink node parameterized by the product of the attention coefficients and the learnable weight parameter, and (2) the aggregated single-message vector from the plurality of source nodes;

determining (312), via the one or more hardware processors, graph level embedding of the molecular graph using a read-out function from the node level embedding of the molecular graph, wherein the read-out function takes node attributes transformed molecular graph as an input and computes a graph-level representation; and

computing (314), via the one or more hardware processors, the one or more molecular properties by feeding the graph level embedding of the molecular graph to a linear layer, wherein the edge-conditioned graph attention neural networks is resilient to noise by learning to adapt to kingpin on a task-relevant fragment of the molecular graphs at varying receptive fields, locality, depth to augment discriminative power of node and graph-level embeddings, the learned discriminative node and the graph-level embeddings aids in the molecular property prediction with a reduced computational complexity.

2. The processor implemented method of claim 1, further comprising:

operating on the molecular graph, a spatial-dynamic neighborhood aggregation-based message passing phase to exchange the plurality of neural messages resulting in transformation and updating of each node level embedding; and

performing statistical average of the corresponding node level embedding obtained from the edge-conditioned graph attention neural network and the spatial-dynamic neighborhood aggregation-based message passing phase.

3. The processor implemented method of claim 1, further comprising:

operating on the molecular graph, a spatial Identity Mapping graph Convolution Networks based message passing phase to exchange the plurality of neural messages resulting in transformation and updating of each node level embedding; and
performing statistical average of the corresponding node level embedding obtained from the edge-conditioned graph attention neural network and the spatial Identity Mapping graph Convolution Networks based message passing phase.

4. The processor implemented method of claim 1, further comprising:

operating on the molecular graph, a spatial-graph attentional propagation based message passing phase to exchange the plurality of neural messages resulting in transformation and updating of each node level embedding; and
performing statistical average of the corresponding node level embedding obtained from the edge-conditioned graph attention neural network and the spatial-graph attentional propagation based message passing phase.

5. The processor implemented method of claim 1, wherein the edge information associated with the edge of the molecular graph comprises edge-type characteristics of the edge and a spatial distance between atoms of molecular graph that are represented as nodes.

6. A system (400) for molecular property prediction using an edge-conditioned graph attention neural networks, comprising:

a memory (415) storing instructions;
one or more communication interfaces (403); and
one or more hardware processors (402) coupled to the memory (415) via the one or more communication interfaces (403), wherein the one or more hardware processors (402) are configured by the instructions to:

access a database comprising a plurality of molecular graphs associated with a plurality of molecules and a plurality of labels indicative of chemical properties of the plurality of the molecular graphs, wherein each molecular graph of the plurality of molecular graphs comprises a plurality of sink nodes, each sink node of the plurality of sink nodes connected to a plurality of source nodes for passing neural messages through a plurality of edges, wherein the edge-conditioned graph attention neural networks allow stacking of layers in which each node in the molecular graph aggregate information over their local-graph neighbors attributes based on molecular graph's connectivity and adaptively adjust to weight nodes of importance;
compute attention coefficients indicative of influence of the plurality of source nodes on a sink node of the plurality of sink nodes based on (1) a softmax output of a matrix product of a learnable attention vector and a concatenated matrix of a linearly transformed hidden state of the sink node and (2) a matrix product of a linearly transformed edge-information associated with an edge connecting the source node from amongst the plurality of source nodes with the sink node, relative difference of hidden state vectors of the source node and the sink node by a learnable weight matrix, wherein the edge-conditioned graph attention neural networks performs self-attention on the nodes of the molecular graph of different sized neighborhood and also involve a shared attentional mechanism across the nodes of the molecular graphs to compute attention coefficients, wherein edge-conditioned graph attention neural networks is agnostic to selection of attention mechanism and operates on variable-sized graph structured inputs;
compute a plurality of neural messages sent from the plurality of source nodes to the sink node as a product of a summation of a set of hidden state vectors of the plurality of source node and the linearly transformed edge-information associated with the edge connecting the source node from amongst the plurality of source nodes with the sink node;
determine a weighted sum of the plurality of neural messages from the plurality of source nodes perceived by the sink node to obtain an aggregated single-message vector from the plurality of source nodes, the weighted sum determined through the linearly transformed edge-information using the attention coefficients;
transform the hidden state vector of the sink node to obtain a node level embedding of the molecular graph, wherein to transform the hidden state vector of the sink node, the one or more hardware processors are configured by the instructions to compute a summation of (1) the linearly transformed hidden state vector of the sink node parameterized by the product of the attention coefficients and the learnable weight parameter, and (2) the aggregated single-message vector from the plurality of source nodes;

determine a graph level embedding of the molecular graph using a read-out function from the node level embedding of the molecular graph, wherein the read-out function takes node attributes transformed molecular graph as an input and computes a graph-level representation; and

compute the one or more molecular properties by feeding the graph level embedding of the molecular graph to a linear layer, wherein the edge-conditioned graph attention neural networks is resilient to noise by learning to adapt to kingpin on a task-relevant fragment of the molecular graphs at varying receptive fields, locality, depth to augment discriminative power of node and graph-level embeddings, the learned discriminative node and the graph-level embeddings aids in the molecular property prediction with a reduced computational complexity.

7. The system of claim 6, wherein the one or more hardware processors are further configured by the instructions to:

operate on the molecular graph, a spatial-dynamic neighborhood aggregation-based message passing phase to exchange the plurality of neural messages resulting in transformation and updating of each node level embedding; and

perform statistical average of the corresponding node level embedding obtained from the edge-conditioned graph attention neural network and the spatial-dynamic neighborhood aggregation-based message passing phase.

8. The system of claim 6, wherein the one or more hardware processors are further configured by the instructions to:

operate on the molecular graph, a spatial Identity Mapping graph Convolution Networks based message passing phase to exchange the plurality of neural messages resulting in transformation and updating of each node level embedding; and

perform statistical average of the corresponding node level embedding obtained from the edge-conditioned graph attention neural network and the spatial Identity Mapping graph Convolution Networks based message passing phase.

9. The system of claim 6, wherein the one or more hardware processors are further configured by the instructions to:

operate on the molecular graph, a spatial-graph attentional propagation based message passing phase to exchange the plurality of neural messages resulting in transformation and updating of each node level embedding; and

perform statistical average of the corresponding node level embedding obtained from the edge-conditioned graph attention neural network and the spatial-graph attentional propagation based message passing phase.

10. The system of claim 6, wherein the edge information associated with the edge of the molecular graph comprises edge-type characteristics of the edge and a spatial distance between atoms of molecular graph that are represented as nodes.

11. One or more non-transitory machine-readable information storage mediums for molecular property prediction using an edge-conditioned graph attention neural networks comprising one or more instructions which when executed by one or more hardware processors cause:

accessing a database comprising a plurality of molecular graphs associated with a plurality of molecules and a plurality of labels indicative of chemical properties of the plurality of the molecular graphs, wherein each molecular graph of the plurality of molecular graphs comprises a plurality of sink nodes, each sink node of the plurality of sink nodes connected to a plurality of source nodes for passing neural messages through a plurality of edges, wherein the edge-conditioned graph attention neural networks allow stacking of layers in which each node in the molecular graph aggregate information over their local-graph neighbors attributes based on molecular graph's connectivity and adaptively adjust to weight nodes of importance;

computing attention coefficients indicative of influence of the plurality of source nodes on a sink node of the plurality of sink nodes based on (1) a softmax output of a matrix product of a learnable attention vector and a concatenated matrix of a linearly transformed hidden state of the sink node, and (2) a matrix product of a linearly transformed edge-information associated with an edge connecting the source node from amongst the plurality of source nodes with the sink node, relative difference of hidden state vectors of the source node and the sink node by a learnable weight matrix, wherein the edge-conditioned graph attention neural networks performs self-attention on the nodes of the molecular graph of different sized neighborhood and also involve a shared attentional mechanism across the nodes of the molecular graphs to compute attention coefficients, wherein edge-conditioned graph attention neural networks is agnostic to selection of attention mechanism and operates on variable-sized graph structured inputs;

computing a plurality of neural messages sent from the plurality of source nodes to the sink node as a product of summation of a set of hidden state vectors of the plurality of source node and the linearly transformed edge-information associated with the edge connecting the source node from amongst the plurality of source nodes with the sink node;

determining a weighted sum of the plurality of neural messages from the plurality of source nodes perceived by the sink node to obtain an aggregated single-message vector from the plurality of source nodes, the weighted sum determined through the linearly transformed edge-information using the attention coefficients;

transforming the hidden state vector of the sink node to obtain a node level embedding of the molecular graph, wherein transforming the hidden state vector of the sink node comprises computing a summation of (1) the linearly transformed hidden state vector of the sink node parameterized by the product of the attention coefficients and the learnable weight parameter, and (2) the aggregated single-message vector from the plurality of source nodes;

determining graph level embedding of the molecular graph using a read-out function from the node level embedding of the molecular graph, wherein the read-out function takes node attributes transformed molecular graph as an input and computes a graph-level representation; and;

computing the one or more molecular properties by feeding the graph level embedding of the molecular graph to a linear layer, wherein the edge-conditioned graph attention neural networks is resilient to noise by learning to adapt to kingpin on a task-relevant fragment of the molecular graphs at varying receptive fields, locality, depth to augment discriminative power of node and graph-level embeddings, the learned discriminative node and the graph-level embeddings aids in the molecular property prediction with a reduced computational complexity.

12. The one or more non-transitory machine readable information storage mediums of claim 11, further comprising:

operating on the molecular graph, a spatial-dynamic neighborhood aggregation-based message passing phase to exchange the plurality of neural messages resulting in transformation and updating of each node level embedding; and

performing statistical average of the corresponding node level embedding obtained from the edge-conditioned graph attention neural network and the spatial-dynamic neighborhood aggregation-based message passing phase.

13. The one or more non-transitory machine readable information storage mediums of claim 11, further comprising:

operating on the molecular graph, a spatial Identity Mapping graph Convolution Networks based message passing phase to exchange the plurality of neural messages resulting in transformation and updating of each node level embedding; and

performing statistical average of the corresponding node level embedding obtained from the edge-conditioned graph attention neural network and the spatial Identity Mapping graph Convolution Networks based message passing phase.

14. The one or more non-transitory machine readable information storage mediums of claim 11, further comprising:

operating on the molecular graph, a spatial-graph attentional propagation based message passing phase to exchange the plurality of neural messages resulting in transformation and updating of each node level embedding; and

performing statistical average of the corresponding node level embedding obtained from the edge-conditioned graph attention neural network and the spatial-graph attentional propagation based message passing phase.

15. The one or more non-transitory machine readable information storage mediums of claim 11, wherein the edge information associated with the edge of the molecular graph comprises edge-type characteristics of the edge and a spatial distance between atoms of molecular graph that are represented as nodes.

**Patentansprüche**

1. Prozessorimplementiertes Verfahren (300) zur molekularen Eigenschaftsvorhersage unter Verwendung eines kantenkonditionierten neuronalen Graphen-Aufmerksamkeitsnetzwerks, umfassend:

   Zugreifen (302), über einen oder mehrere Hardwareprozessoren, auf eine Datenbank, umfassend eine Mehrzahl von molekularen Graphen, die einer Mehrzahl von Molekülen zugeordnet sind, und eine Mehrzahl von Markierungen, die chemische Eigenschaften der Mehrzahl der molekularen Graphen anzeigen, wobei jeder molekulare Graph der Mehrzahl von molekularen Graphen eine Mehrzahl von Senkenknoten umfasst, wobei jeder Senkenknoten der Mehrzahl von Senkenknoten mit einer Mehrzahl von Quellenknoten verbunden ist, um neuronale Nachrichten durch eine Mehrzahl von Kanten zu leiten, wobei die kantenkonditionierten neuronalen Graphen-Aufmerksamkeitsnetzwerke ein Stapeln von Schichten ermöglichen, in denen jeder Knoten in dem molekularen Graphen Informationen über seine Lokalgraphen-Nachbarattribute basierend auf der Konnektivität des molekularen Graphen aggregiert und sich adaptiv an wichtige Gewichtsknoten anpasst;
   Berechnen (304), über den einen oder die mehreren Hardwareprozessoren, von Aufmerksamkeitskoeffizienten, die einen Einfluss der Mehrzahl von Quellenknoten auf einen Senkenknoten der Mehrzahl von Senkenknoten anzeigen, basierend auf (1) einer Softmax-Ausgabe eines Matrixprodukts eines lernbaren Aufmerksamkeitsvektors und einer verketteten Matrix eines linear transformierten verborgenen Zustands des Senkenknotens, und (2) einem Matrixprodukt einer linear transformierten Kanteninformation, die einer Kante zugeordnet ist, die den Quellenknoten aus der Mehrzahl von Quellenknoten mit dem Senkenknoten verbindet, relativer Differenz von verborgenen Zustandsvektoren des Quellenknotens und des Senkenknotens durch eine lernbare Gewichtsmatrix, wobei die kantenkonditionierten neuronalen Graphen-Aufmerksamkeitsnetzwerke eine Selbstaufmerksamkeit an den Knoten des molekularen Graphen unterschiedlich großer Nachbarschaft durchführen und auch einen gemeinsamen Aufmerksamkeitsmechanismus über die Knoten der molekularen Graphen hinweg einbeziehen, um Aufmerksamkeitskoeffizienten zu berechnen, wobei die kantenkonditionierten neuronalen Graphen-Aufmerksamkeitsnetzwerke für die Auswahl des Aufmerksamkeitsmechanismus agnostisch sind und auf graphenstrukturierten Eingaben variabler Größe arbeiten;
   Berechnen (306), über den einen oder die mehreren Hardwareprozessoren, einer Mehrzahl von neuronalen Nachrichten, die von der Mehrzahl von Quellenknoten an den Senkenknoten gesendet werden, als ein Produkt einer Summierung eines Satzes von verborgenen Zustandsvektoren der Mehrzahl von Quellenknoten und der linear transformierten Kanteninformation, die der Kante zugeordnet ist, die den Quellenknoten aus der Mehrzahl von Quellenknoten mit dem Senkenknoten verbindet;
   Bestimmen (308), über den einen oder die mehreren Hardwareprozessoren, einer gewichteten Summe der Mehrzahl von neuronalen Nachrichten von der Mehrzahl von Quellenknoten, die von dem Senkenknoten wahrgenommen werden, um einen aggregierten Einzelnachrichtenvektor von der Mehrzahl von Quellenknoten zu erhalten, wobei die gewichtete Summe durch die linear transformierte Kanteninformation unter Verwendung der Aufmerksamkeitskoeffizienten bestimmt wird;
   Transformieren (310), über den einen oder die mehreren Hardwareprozessoren, des verborgenen Zustandsvektors des Senkenknotens, um eine Einbettung auf Knotenebene des molekularen Graphen zu erhalten, wobei das Transformieren des verborgenen Zustandsvektors des Senkenknotens ein Berechnen einer Summierung von (1) dem linear transformierten verborgenen Zustandsvektor des Senkenknotens, der durch das Produkt der Aufmerksamkeitskoeffizienten und des lernbaren Gewichtsparameters parametrisiert ist, und (2) dem aggregierten Einzelnachrichtenvektor von der Mehrzahl von Quellenknoten umfasst;
   Bestimmen (312), über den einen oder die mehreren Hardwareprozessoren, einer Einbettung auf Graphenebene des molekularen Graphen unter Verwendung einer Auslesefunktion aus der Einbettung auf Knotenebene des molekularen Graphen, wobei die Auslesefunktion Knotenattribute des transformierten molekularen Graphen als eine Eingabe nimmt und eine Darstellung auf Graphenebene berechnet; und
   Berechnen (314), über den einen oder die mehreren Hardwareprozessoren, der einen oder der mehreren molekularen Eigenschaften durch Einspeisen der Einbettung auf Graphenebene des molekularen Graphen in eine lineare Schicht, wobei die kantenkonditionierten neuronalen Graphen-Aufmerksamkeitsnetzwerke gegenüber Rauschen widerstandsfähig sind, indem sie lernen, sich an Kingspin an einem aufgabenrelevanten Fragment der molekularen Graphen bei variierenden Aufnahmefeldern, Lokalität, Tiefe anzupassen, um die Unterscheidungskraft von Einbettungen auf Knoten- und Graphenebene zu erhöhen, wobei der gelernte Unterscheidungsknoten und die Einbettungen auf Graphenebene bei der molekularen Eigenschaftsvorhersage mit einer reduzierten Rechenkomplexität helfen.

2. Prozessorimplementiertes Verfahren nach Anspruch 1, ferner umfassend:

Betreiben, an dem molekularen Graphen, einer auf räumlich-dynamischer Nachbarschaftsaggregation basierenden Nachrichtendurchgangsphase, um die Mehrzahl von neuronalen Nachrichten auszutauschen, was zu einer Transformation und Aktualisierung jeder Einbettung auf Knotenebene führt; und

Durchführen eines statistischen Durchschnitts der entsprechenden Einbettung auf Knotenebene, die von dem kantenkonditionierten neuronalen Graphen-Aufmerksamkeitsnetzwerk und der auf räumlich-dynamischer Nachbarschaftsaggregation basierenden Nachrichtendurchgangsphase erhalten wird.

3. Prozessorimplementiertes Verfahren nach Anspruch 1, ferner umfassend:

Betreiben, an dem molekularen Graphen, einer auf räumlichem Identitätsabbildungsgraphen-Faltungsnetzwerken basierenden Nachrichtendurchgangsphase, um die Mehrzahl von neuronalen Nachrichten auszutauschen, was zu einer Transformation und Aktualisierung jeder Einbettung auf Knotenebene führt; und

Durchführen eines statistischen Durchschnitts der entsprechenden Einbettung auf Knotenebene, die von dem kantenkonditionierten neuronalen Graphen-Aufmerksamkeitsnetzwerk und der auf räumlichem Identitätsabbildungsgraphen-Faltungsnetzwerken basierenden Nachrichtendurchgangsphase erhalten wird.

4. Prozessorimplementiertes Verfahren nach Anspruch 1, ferner umfassend:

Betreiben, an dem molekularen Graphen, einer auf räumlicher Graphen-Aufmerksamkeitsausbreitung basierenden Nachrichtendurchgangsphase, um die Mehrzahl von neuronalen Nachrichten auszutauschen, was zu einer Transformation und Aktualisierung jeder Einbettung auf Knotenebene führt; und

Durchführen eines statistischen Durchschnitts der entsprechenden Einbettung auf Knotenebene, die von dem kantenkonditionierten neuronalen Graphen-Aufmerksamkeitsnetzwerk und der auf räumlicher Graphen-Aufmerksamkeitsausbreitung basierenden Nachrichtendurchgangsphase erhalten wird.

5. Prozessorimplementiertes Verfahren nach Anspruch 1, wobei die Kanteninformationen, die mit der Kante des molekularen Graphen assoziiert sind, Kantentypeigenschaften der Kante und einen räumlichen Abstand zwischen Atomen des molekularen Graphen, die als Knoten dargestellt sind, umfassen.

6. System (400) zur molekularen Eigenschaftsvorhersage unter Verwendung eines kantenkonditionierten neuronalen Graphen-Aufmerksamkeitsnetzwerks, umfassend:

einen Speicher (415), der Anweisungen speichert;
eine oder mehrere Kommunikationsschnittstellen (403); und
einen oder mehrere Hardwareprozessoren (402), die über die eine oder die mehreren Kommunikationsschnittstellen (403) mit dem Speicher (415) gekoppelt sind, wobei der eine oder die mehreren Hardwareprozessoren (402) durch die Anweisungen konfiguriert sind zum:

Zugreifen auf eine Datenbank, umfassend eine Mehrzahl von molekularen Graphen, die einer Mehrzahl von Molekülen zugeordnet sind, und eine Mehrzahl von Markierungen, die chemische Eigenschaften der Mehrzahl der molekularen Graphen anzeigen, wobei jeder molekulare Graph der Mehrzahl von molekularen Graphen eine Mehrzahl von Senkenknoten umfasst, wobei jeder Senkenknoten der Mehrzahl von Senkenknoten mit einer Mehrzahl von Quellenknoten verbunden ist, um neuronale Nachrichten durch eine Mehrzahl von Kanten zu leiten, wobei die kantenkonditionierten neuronalen Graphen-Aufmerksamkeitsnetzwerke ein Stapeln von Schichten ermöglichen, in denen jeder Knoten in dem molekularen Graphen Informationen über seine Lokalgraphen-Nachbarnattribute basierend auf der Konnektivität des molekularen Graphen aggregiert und sich adaptiv an wichtige Gewichtsknoten anpasst;
Berechnen von Aufmerksamkeitskoeffizienten, die einen Einfluss der Mehrzahl von Quellenknoten auf einen Senkenknoten der Mehrzahl von Senkenknoten anzeigen, basierend auf (1) einer Softmax-Ausgabe eines Matrixprodukts eines lernbaren Aufmerksamkeitsvektors und einer verketteten Matrix eines linear transformierten verborgenen Zustands des Senkenknotens, und (2) einem Matrixprodukt einer linear transformierten Kanteninformation, die einer Kante zugeordnet ist, die den Quellenknoten aus der Mehrzahl von Quellenknoten mit dem Senkenknoten verbindet, relativer Differenz von verborgenen Zustandsvektoren des Quellenknotens und des Senkenknotens durch eine lernbare Gewichtsmatrix, wobei die kantenkonditionierten neuronalen Graphen-Aufmerksamkeitsnetzwerke eine Selbstaufmerksamkeit an den Knoten des molekularen Graphen unterschiedlich großer Nachbarschaft durchführen und auch einen gemeinsamen Aufmerksamkeitsmechanismus über die Knoten der molekularen Graphen hinweg einbeziehen, um Aufmerksamkeitskoeffizienten zu berechnen, wobei die kantenkonditionierten neuronalen Graphen-Aufmerksamkeitsnetzwerke für die Auswahl des Aufmerksamkeitsmechanismus agnostisch sind und auf graphenstrukturierten Eingaben variabler Größe arbeiten;

Berechnen einer Mehrzahl von neuronalen Nachrichten, die von der Mehrzahl von Quellenknoten an den Senkenknoten gesendet werden, als ein Produkt einer Summierung eines Satzes von verborgenen Zustandsvektoren der Mehrzahl von Quellenknoten und der linear transformierten Kanteninformation, die der Kante zugeordnet ist, die den Quellenknoten aus der Mehrzahl von Quellenknoten mit dem Senkenknoten verbindet;

Bestimmen einer gewichteten Summe der Mehrzahl von neuronalen Nachrichten von der Mehrzahl von Quellenknoten, die von dem Senkenknoten wahrgenommen werden, um einen aggregierten Einzelnachrichtenvektor von der Mehrzahl von Quellenknoten zu erhalten, wobei die gewichtete Summe durch die linear transformierte Kanteninformation unter Verwendung der Aufmerksamkeitskoeffizienten bestimmt wird;

Transformieren des verborgenen Zustandsvektors des Senkenknotens, um eine Einbettung auf Knotenebene des molekularen Graphen zu erhalten, wobei zum Transformieren des verborgenen Zustandsvektors des Senkenknotens der eine oder die mehreren Hardwareprozessoren durch die Anweisungen konfiguriert sind zum Berechnen einer Summierung von (1) dem linear transformierten verborgenen Zustandsvektor des Senkenknotens, der durch das Produkt der Aufmerksamkeitskoeffizienten und des lernbaren Gewichtsparameters parametrisiert ist, und (2) dem aggregierten Einzelnachrichtenvektor von der Mehrzahl von Quellenknoten;

Bestimmen einer Einbettung auf Graphenebene des molekularen Graphen unter Verwendung einer Auslesefunktion aus der Einbettung auf Knotenebene des molekularen Graphen, wobei die Auslesefunktion Knotenattribute des transformierten molekularen Graphen als eine Eingabe nimmt und eine Darstellung auf Graphenebene berechnet; und

Berechnen der einen oder der mehreren molekularen Eigenschaften durch Einspeisen der Einbettung auf Graphenebene des molekularen Graphen in eine lineare Schicht, wobei die kantenkonditionierten neuronalen Graphen-Aufmerksamkeitsnetzwerke gegenüber Rauschen widerstandsfähig sind, indem sie lernen, sich an Kingspin an einem aufgabenrelevanten Fragment der molekularen Graphen bei variierenden Aufnahmefeldern, Lokalität, Tiefe anzupassen, um die Unterscheidungskraft von Einbettungen auf Knoten- und Graphenebene zu erhöhen, wobei der gelernte Unterscheidungsknoten und die Einbettungen auf Graphenebene bei der molekularen Eigenschaftsvorhersage mit einer reduzierten Rechenkomplexität helfen.

7. System nach Anspruch 6, wobei der eine oder die mehreren Hardwareprozessoren ferner durch die Anweisungen konfiguriert sind zum:

Betreiben, an dem molekularen Graphen, einer auf räumlich-dynamischer Nachbarschaftsaggregation basierenden Nachrichtendurchgangsphase, um die Mehrzahl von neuronalen Nachrichten auszutauschen, was zu einer Transformation und Aktualisierung jeder Einbettung auf Knotenebene führt; und

Durchführen eines statistischen Durchschnitts der entsprechenden Einbettung auf Knotenebene, die von dem kantenkonditionierten neuronalen Graphen-Aufmerksamkeitsnetzwerk und der auf räumlich-dynamischer Nachbarschaftsaggregation basierenden Nachrichtendurchgangsphase erhalten wird.

8. System nach Anspruch 6, wobei der eine oder die mehreren Hardwareprozessoren ferner durch die Anweisungen konfiguriert sind zum:

Betreiben, an dem molekularen Graphen, einer auf räumlichem Identitätsabbildungsgraphen-Faltungsnetzwerken basierenden Nachrichtendurchgangsphase, um die Mehrzahl von neuronalen Nachrichten auszutauschen, was zu einer Transformation und Aktualisierung jeder Einbettung auf Knotenebene führt; und

Durchführen eines statistischen Durchschnitts der entsprechenden Einbettung auf Knotenebene, die von dem kantenkonditionierten neuronalen Graphen-Aufmerksamkeitsnetzwerk und der auf räumlichem Identitätsabbildungsgraphen-Faltungsnetzwerken basierenden Nachrichtendurchgangsphase erhalten wird.

9. System nach Anspruch 6, wobei der eine oder die mehreren Hardwareprozessoren ferner durch die Anweisungen konfiguriert sind zum:

Betreiben, an dem molekularen Graphen, einer auf räumlicher Graphen-Aufmerksamkeitsausbreitung basierenden Nachrichtendurchgangsphase, um die Mehrzahl von neuronalen Nachrichten auszutauschen, was zu einer Transformation und Aktualisierung jeder Einbettung auf Knotenebene führt; und

Durchführen eines statistischen Durchschnitts der entsprechenden Einbettung auf Knotenebene, die von dem kantenkonditionierten neuronalen Graphen-Aufmerksamkeitsnetzwerk und der auf räumlicher Graphen-Aufmerksamkeitsausbreitung basierenden Nachrichtendurchgangsphase erhalten wird.

10. System nach Anspruch 6, wobei die Kanteninformationen, die mit der Kante des molekularen Graphen assoziiert sind, Kantentypeigenschaften der Kante und einen räumlichen Abstand zwischen Atomen des molekularen Graphen, die als Knoten dargestellt sind, umfassen.

11. Ein oder mehrere nichtflüchtige maschinenlesbare Informationsspeichermedien zur molekularen Eigenschaftsvorhersage unter Verwendung eines kantenkonditionierten neuronalen Graphen-Aufmerksamkeitsnetzwerks, umfassend eine oder mehrere Anweisungen, die bei Ausführung durch einen oder mehrere Hardwareprozessoren bewirken:

Zugreifen auf eine Datenbank, umfassend eine Mehrzahl von molekularen Graphen, die einer Mehrzahl von Molekülen zugeordnet sind, und eine Mehrzahl von Markierungen, die chemische Eigenschaften der Mehrzahl der molekularen Graphen anzeigen, wobei jeder molekulare Graph der Mehrzahl von molekularen Graphen eine Mehrzahl von Senkenknoten umfasst, wobei jeder Senkenknoten der Mehrzahl von Senkenknoten mit einer Mehrzahl von Quellenknoten verbunden ist, um neuronale Nachrichten durch eine Mehrzahl von Kanten zu leiten, wobei die kantenkonditionierten neuronalen Graphen-Aufmerksamkeitsnetzwerke ein Stapeln von Schichten ermöglichen, in denen jeder Knoten in dem molekularen Graphen Informationen über seine Lokalgraphen-Nachbarnattribute basierend auf der Konnektivität des molekularen Graphen aggregiert und sich adaptiv an wichtige Gewichtsknoten anpasst;
Berechnen von Aufmerksamkeitskoeffizienten, die einen Einfluss der Mehrzahl von Quellenknoten auf einen Senkenknoten der Mehrzahl von Senkenknoten anzeigen, basierend auf (1) einer Softmax-Ausgabe eines Matrixprodukts eines lernbaren Aufmerksamkeitsvektors und einer verketteten Matrix eines linear transformierten verborgenen Zustands des Senkenknotens, und (2) einem Matrixprodukt einer linear transformierten Kanteninformation, die einer Kante zugeordnet ist, die den Quellenknoten aus der Mehrzahl von Quellenknoten mit dem Senkenknoten verbindet, relativer Differenz von verborgenen Zustandsvektoren des Quellenknotens und des Senkenknotens durch eine lernbare Gewichtsmatrix, wobei die kantenkonditionierten neuronalen Graphen-Aufmerksamkeitsnetzwerke eine Selbstaufmerksamkeit an den Knoten des molekularen Graphen unterschiedlich großer Nachbarschaft durchführen und auch einen gemeinsamen Aufmerksamkeitsmechanismus über die Knoten der molekularen Graphen hinweg einbeziehen, um Aufmerksamkeitskoeffizienten zu berechnen, wobei die kantenkonditionierten neuronalen Graphen-Aufmerksamkeitsnetzwerke für die Auswahl des Aufmerksamkeitsmechanismus agnostisch sind und auf graphenstrukturierten Eingaben variabler Größe arbeiten;
Berechnen einer Mehrzahl von neuronalen Nachrichten, die von der Mehrzahl von Quellenknoten an den Senkenknoten gesendet werden, als ein Produkt einer Summierung eines Satzes von verborgenen Zustandsvektoren der Mehrzahl von Quellenknoten und der linear transformierten Kanteninformation, die der Kante zugeordnet ist, die den Quellenknoten aus der Mehrzahl von Quellenknoten mit dem Senkenknoten verbindet;
Bestimmen einer gewichteten Summe der Mehrzahl von neuronalen Nachrichten von der Mehrzahl von Quellenknoten, die von dem Senkenknoten wahrgenommen werden, um einen aggregierten Einzelnachrichtenvektor von der Mehrzahl von Quellenknoten zu erhalten, wobei die gewichtete Summe durch die linear transformierte Kanteninformation unter Verwendung der Aufmerksamkeitskoeffizienten bestimmt wird;
Transformieren des verborgenen Zustandsvektors des Senkenknotens, um eine Einbettung auf Knotenebene des molekularen Graphen zu erhalten, wobei das Transformieren des verborgenen Zustandsvektors des Senkenknotens ein Berechnen einer Summierung von (1) dem linear transformierten verborgenen Zustandsvektor des Senkenknotens, der durch das Produkt der Aufmerksamkeitskoeffizienten und des lernbaren Gewichtsparameters parametrisiert ist, und (2) dem aggregierten Einzelnachrichtenvektor von der Mehrzahl von Quellenknoten umfasst;
Bestimmen einer Einbettung auf Graphenebene des molekularen Graphen unter Verwendung einer Auslesefunktion aus der Einbettung auf Knotenebene des molekularen Graphen, wobei die Auslesefunktion Knotenattribute des transformierten molekularen Graphen als eine Eingabe nimmt und eine Darstellung auf Graphenebene berechnet; und
Berechnen der einen oder der mehreren molekularen Eigenschaften durch Einspeisen der Einbettung auf Graphenebene des molekularen Graphen in eine lineare Schicht, wobei die kantenkonditionierten neuronalen Graphen-Aufmerksamkeitsnetzwerke gegenüber Rauschen widerstandsfähig sind, indem sie lernen, sich an Kingspin an einem aufgabenrelevanten Fragment der molekularen Graphen bei variierenden Aufnahmefeldern, Lokalität, Tiefe anzupassen, um die Unterscheidungskraft von Einbettungen auf Knoten- und Graphenebene zu erhöhen, wobei der gelernte Unterscheidungsknoten und die Einbettungen auf Graphenebene bei der molekularen Eigenschaftsvorhersage mit einer reduzierten Rechenkomplexität helfen.

**12.** Ein oder mehrere nichtflüchtige maschinenlesbare Informationsspeichermedien nach Anspruch 11, ferner umfassend:

Betreiben, an dem molekularen Graphen, einer auf räumlich-dynamischer Nachbarschaftsaggregation basierenden Nachrichtendurchgangsphase, um die Mehrzahl von neuronalen Nachrichten auszutauschen, was zu einer Transformation und Aktualisierung jeder Einbettung auf Knotenebene führt; und
Durchführen eines statistischen Durchschnitts der entsprechenden Einbettung auf Knotenebene, die von dem kantenkonditionierten neuronalen Graphen-Aufmerksamkeitsnetzwerk und der auf räumlich-dynamischer Nachbarschaftsaggregation basierenden Nachrichtendurchgangsphase erhalten wird.

**13.** Ein oder mehrere nichtflüchtige maschinenlesbare Informationsspeichermedien nach Anspruch 11, ferner umfassend:

Betreiben, an dem molekularen Graphen, einer auf räumlichem Identitätsabbildungsgraphen-Faltungsnetzwerken basierenden Nachrichtendurchgangsphase, um die Mehrzahl von neuronalen Nachrichten auszutauschen, was zu einer Transformation und Aktualisierung jeder Einbettung auf Knotenebene führt; und
Durchführen eines statistischen Durchschnitts der entsprechenden Einbettung auf Knotenebene, die von dem kantenkonditionierten neuronalen Graphen-Aufmerksamkeitsnetzwerk und der auf räumlichem Identitätsabbildungsgraphen-Faltungsnetzwerken basierenden Nachrichtendurchgangsphase erhalten wird.

**14.** Ein oder mehrere nichtflüchtige maschinenlesbare Informationsspeichermedien nach Anspruch 11, ferner umfassend:

Betreiben, an dem molekularen Graphen, einer auf räumlicher Graphen-Aufmerksamkeitsausbreitung basierenden Nachrichtendurchgangsphase, um die Mehrzahl von neuronalen Nachrichten auszutauschen, was zu einer Transformation und Aktualisierung jeder Einbettung auf Knotenebene führt; und
Durchführen eines statistischen Durchschnitts der entsprechenden Einbettung auf Knotenebene, die von dem kantenkonditionierten neuronalen Graphen-Aufmerksamkeitsnetzwerk und der auf räumlicher Graphen-Aufmerksamkeitsausbreitung basierenden Nachrichtendurchgangsphase erhalten wird.

**15.** Ein oder mehrere nichtflüchtige maschinenlesbare Informationsspeichermedien nach Anspruch 11, wobei die Kanteninformationen, die mit der Kante des molekularen Graphen assoziiert sind, Kantentypeigenschaften der Kante und einen räumlichen Abstand zwischen Atomen des molekularen Graphen, die als Knoten dargestellt sind, umfassen.

## Revendications

**1.** Procédé mis en œuvre par processeur (300) pour la prédiction de propriétés moléculaires en utilisant des réseaux neuronaux d'attention de graphe conditionné par les arêtes, comprenant :

l'accès (302), via un ou plusieurs processeurs matériels, à une base de données comprenant une pluralité de graphes moléculaires associés à une pluralité de molécules et une pluralité d'étiquettes indicatives de propriétés chimiques de la pluralité des graphes moléculaires, dans lequel chaque graphe moléculaire de la pluralité de graphes moléculaires comprend une pluralité de noeuds cible, chaque noeud cible de la pluralité de noeuds cible étant connecté à une pluralité de noeuds sources pour faire passer des messages neuronaux à travers une pluralité d'arêtes, dans lequel les réseaux neuronaux d'attention de graphe conditionné par les arêtes permettent l'empilement de couches dans lesquelles chaque noeud dans le graphe moléculaire agrège des informations sur ses attributs de voisins de graphe local sur la base de la connectivité du graphe moléculaire et s'ajuste de manière adaptative à des noeuds de poids d'importance ;
le calcul (304), via les un ou plusieurs processeurs matériels, de coefficients d'attention indicatifs d'une influence de la pluralité de noeuds sources sur un noeud cible de la pluralité de noeuds cible sur la base (1) d'une sortie softmax S d'un produit matriciel d'un vecteur d'attention pouvant être appris et d'une matrice concaténée d'un état caché transformé linéairement du noeud cible, et (2) d'un produit matriciel d'informations d'arête transformées linéairement associées à une arête connectant le noeud source parmi la pluralité de noeuds sources au noeud cible, d'une différence relative de vecteurs d'état cachés du noeud source et du noeud cible par une matrice de poids pouvant être apprise, dans lequel les réseaux neuronaux d'attention de graphe conditionné par les arêtes effectuent une auto-attention sur les noeuds du graphe moléculaire de voisinage de taille différente et impliquent également un mécanisme d'attention partagé à travers les noeuds des graphes moléculaires pour calculer des coefficients d'attention, dans lequel les réseaux neuronaux d'attention de graphe conditionné par les arêtes sont agnostiques à la sélection de mécanisme d'attention et fonctionnent sur des entrées structurées de graphe de taille variable ;

le calcul (306), via les un ou plusieurs processeurs matériels, d'une pluralité de messages neuronaux envoyés de la pluralité de noeuds sources au noeud cible en tant que produit d'une sommation d'un ensemble de vecteurs d'état cachés de la pluralité de noeuds sources et des informations d'arête transformées linéairement associées à l'arête connectant le noeud source parmi la pluralité de noeuds sources au noeud cible ;

la détermination (308), via les un ou plusieurs processeurs matériels, d'une somme pondérée de la pluralité de messages neuronaux provenant de la pluralité de noeuds sources perçus par le noeud cible pour obtenir un vecteur de message unique agrégé à partir de la pluralité de noeuds sources, la somme pondérée étant déterminée par le biais des informations d'arête transformées linéairement en utilisant les coefficients d'attention ;

la transformation (310), via les un ou plusieurs processeurs matériels, du vecteur d'état caché du noeud cible pour obtenir une intégration de niveau de noeud du graphe moléculaire, dans lequel la transformation du vecteur d'état caché du noeud cible comprend le calcul d'une sommation (1) du vecteur d'état caché transformé linéairement du noeud cible paramétré par le produit des coefficients d'attention et du paramètre de poids pouvant être appris, et (2) du vecteur de message unique agrégé à partir de la pluralité de noeuds sources ;

la détermination (312), via les un ou plusieurs processeurs matériels, d'une intégration de niveau de graphe du graphe moléculaire en utilisant une fonction de lecture à partir de l'intégration de niveau de noeud du graphe moléculaire, dans lequel la fonction de lecture prend un graphe moléculaire transformé par attributs de noeud en tant qu'entrée et calcule une représentation de niveau de graphe ; et

le calcul (314), via les un ou plusieurs processeurs matériels, des une ou plusieurs propriétés moléculaires en alimentant l'intégration de niveau de graphe du graphe moléculaire à une couche linéaire, dans lequel les réseaux neuronaux d'attention de graphe conditionné par les arêtes sont résilients au bruit par apprentissage pour s'adapter à l'élément pivot sur un fragment pertinent pour une tâche des graphes moléculaires à des champs réceptifs variables, une localité, une profondeur pour augmenter une puissance discriminante d'intégrations de noeud et de niveau de graphe, le noeud discriminant appris et les intégrations de niveau de graphe aident à la prédiction de propriétés moléculaires avec une complexité de calcul réduite.

2. Procédé mis en œuvre par processeur selon la revendication 1, comprenant en outre :

le fonctionnement sur le graphe moléculaire d'une phase de passage de message basée sur une agrégation de voisinage spatialement dynamique pour échanger la pluralité de messages neuronaux résultant en une transformation et une mise à jour de chaque intégration de niveau de noeud ; et

la réalisation d'une moyenne statistique de l'intégration de niveau de noeud correspondante obtenue à partir du réseau neuronal d'attention de graphe conditionné par les arêtes et de la phase de passage de message basée sur une agrégation de voisinage spatialement dynamique.

3. Procédé mis en œuvre par processeur selon la revendication 1, comprenant en outre :

le fonctionnement sur le graphe moléculaire d'une phase de passage de message basée sur des réseaux de convolution de graphe de mappage d'identité spatial pour échanger la pluralité de messages neuronaux résultant en une transformation et une mise à jour de chaque intégration de niveau de noeud ; et

la réalisation d'une moyenne statistique de l'intégration de niveau de noeud correspondante obtenue à partir du réseau neuronal d'attention de graphe conditionné par les arêtes et de la phase de passage de message basée sur des réseaux de convolution de graphe de mappage d'identité spatial.

4. Procédé mis en œuvre par processeur selon la revendication 1, comprenant en outre :

le fonctionnement sur le graphe moléculaire d'une phase de passage de message basée sur une propagation attentionnelle de graphe spatial pour échanger la pluralité de messages neuronaux résultant en une transformation et une mise à jour de chaque intégration de niveau de noeud ; et

la réalisation d'une moyenne statistique de l'intégration de niveau de noeud correspondante obtenue à partir du réseau neuronal d'attention de graphe conditionné par les arêtes et de la phase de passage de message basée sur une propagation attentionnelle de graphe spatial.

5. Procédé mis en œuvre par processeur selon la revendication 1, dans lequel les informations d'arête associées à l'arête du graphe moléculaire comprennent des caractéristiques de type d'arête de l'arête et une distance spatiale entre des atomes de graphe moléculaire qui sont représentés en tant que noeuds.

6. Système (400) pour la prédiction de propriétés moléculaires en utilisant des réseaux neuronaux d'attention de graphe conditionné par les arêtes, comprenant :

une mémoire (415) stockant des instructions ;
une ou plusieurs interfaces de communication (403) ; et
un ou plusieurs processeurs matériels (402) couplés à la mémoire (415) via les une ou plusieurs interfaces de communication (403), dans lequel les un ou plusieurs processeurs matériels (402) sont configurés par les instructions pour :

accéder à une base de données comprenant une pluralité de graphes moléculaires associés à une pluralité de molécules et une pluralité d'étiquettes indicatives de propriétés chimiques de la pluralité des graphes moléculaires, dans lequel chaque graphe moléculaire de la pluralité de graphes moléculaires comprend une pluralité de noeuds cible, chaque noeud cible de la pluralité de noeuds cible étant connecté à une pluralité de noeuds sources pour faire passer des messages neuronaux à travers une pluralité d'arêtes, dans lequel les réseaux neuronaux d'attention de graphe conditionné par les arêtes permettent l'empilement de couches dans lesquelles chaque noeud dans le graphe moléculaire agrège des informations sur ses attributs de voisins de graphe local sur la base de la connectivité du graphe moléculaire et s'ajuste de manière adaptative à des noeuds de poids d'importance ;
calculer des coefficients d'attention indicatifs d'une influence de la pluralité de noeuds sources sur un noeud cible de la pluralité de noeuds cible sur la base (1) d'une sortie softmax d'un produit matriciel d'un vecteur d'attention pouvant être appris et d'une matrice concaténée d'un état caché transformé linéairement du noeud cible et (2) d'un produit matriciel d'informations d'arête transformées linéairement associées à une arête connectant le noeud source parmi la pluralité de noeuds sources au noeud cible, d'une différence relative de vecteurs d'état cachés du noeud source et du noeud cible par une matrice de poids pouvant être apprise, dans lequel les réseaux neuronaux d'attention de graphe conditionné par les arêtes effectuent une auto-attention sur les noeuds du graphe moléculaire de voisinage de taille différente et impliquent également un mécanisme d'attention partagé à travers les noeuds des graphes moléculaires pour calculer des coefficients d'attention, dans lequel les réseaux neuronaux d'attention de graphe conditionné par les arêtes sont agnostiques à la sélection de mécanisme d'attention et fonctionnent sur des entrées structurées de graphe de taille variable ;
calculer une pluralité de messages neuronaux envoyés de la pluralité de noeuds sources au noeud cible en tant que produit d'une sommation d'un ensemble de vecteurs d'état cachés de la pluralité de noeuds sources et des informations d'arête transformées linéairement associées à l'arête connectant le noeud source parmi la pluralité de noeuds sources au noeud cible ;
déterminer une somme pondérée de la pluralité de messages neuronaux provenant de la pluralité de noeuds sources perçus par le noeud cible pour obtenir un vecteur de message unique agrégé à partir de la pluralité de noeuds sources, la somme pondérée étant déterminée par le biais des informations d'arête transformées linéairement en utilisant les coefficients d'attention ;
transformer le vecteur d'état caché du noeud cible pour obtenir une intégration de niveau de noeud du graphe moléculaire, dans lequel pour transformer le vecteur d'état caché du noeud cible, les un ou plusieurs processeurs matériels sont configurés par les instructions pour calculer une sommation (1) du vecteur d'état caché transformé linéairement du noeud cible paramétré par le produit des coefficients d'attention et du paramètre de poids pouvant être appris, et (2) du vecteur de message unique agrégé à partir de la pluralité de noeuds sources ;
déterminer une intégration de niveau de graphe du graphe moléculaire en utilisant une fonction de lecture à partir de l'intégration de niveau de noeud du graphe moléculaire, dans lequel la fonction de lecture prend un graphe moléculaire transformé par attributs de noeud en tant qu'entrée et calcule une représentation de niveau de graphe ; et
calculer les une ou plusieurs propriétés moléculaires en alimentant l'intégration de niveau de graphe du graphe moléculaire à une couche linéaire, dans lequel les réseaux neuronaux d'attention de graphe conditionné par les arêtes sont résilients au bruit par apprentissage pour s'adapter à l'élément pivot sur un fragment pertinent pour une tâche des graphes moléculaires à des champs réceptifs variables, une localité, une profondeur pour augmenter une puissance discriminante d'intégrations de noeud et de niveau de graphe, le noeud discriminant appris et les intégrations de niveau de graphe aident à la prédiction de propriétés moléculaires avec une complexité de calcul réduite.

7. Système selon la revendication 6, dans lequel les un ou plusieurs processeurs matériels sont en outre configurés par les instructions pour :

le fonctionnement sur le graphe moléculaire d'une phase de passage de message basée sur une agrégation de voisinage spatialement dynamique pour échanger la pluralité de messages neuronaux résultant en une transformation et une mise à jour de chaque intégration de niveau de noeud ; et
la réalisation d'une moyenne statistique de l'intégration de niveau de noeud correspondante obtenue à partir du réseau neuronal d'attention de graphe conditionné par les arêtes et de la phase de passage de message basée sur une agrégation de voisinage spatialement dynamique.

8. Système selon la revendication 6, dans lequel les un ou plusieurs processeurs matériels sont en outre configurés par les instructions pour :

le fonctionnement sur le graphe moléculaire d'une phase de passage de message basée sur des réseaux de convolution de graphe de mappage d'identité spatial pour échanger la pluralité de messages neuronaux résultant en une transformation et une mise à jour de chaque intégration de niveau de noeud ; et
la réalisation d'une moyenne statistique de l'intégration de niveau de noeud correspondante obtenue à partir du réseau neuronal d'attention de graphe conditionné par les arêtes et de la phase de passage de message basée sur des réseaux de convolution de graphe de mappage d'identité spatial.

9. Système selon la revendication 6, dans lequel les un ou plusieurs processeurs matériels sont en outre configurés par les instructions pour :

le fonctionnement sur le graphe moléculaire d'une phase de passage de message basée sur une propagation attentionnelle de graphe spatial pour échanger la pluralité de messages neuronaux résultant en une transformation et une mise à jour de chaque intégration de niveau de noeud ; et
la réalisation d'une moyenne statistique de l'intégration de niveau de noeud correspondante obtenue à partir du réseau neuronal d'attention de graphe conditionné par les arêtes et de la phase de passage de message basée sur une propagation attentionnelle de graphe spatial.

10. Système selon la revendication 6, dans lequel les informations d'arête associées à l'arête du graphe moléculaire comprennent des caractéristiques de type d'arête de l'arête et une distance spatiale entre des atomes de graphe moléculaire qui sont représentés en tant que noeuds.

11. Un ou plusieurs supports de stockage d'informations lisibles par machine non transitoires pour la prédiction de propriétés moléculaires en utilisant des réseaux neuronaux d'attention de graphe conditionné par les arêtes comprenant une ou plusieurs instructions qui, lorsqu'elles sont exécutées par un ou plusieurs processeurs matériels, amènent :

l'accès à une base de données comprenant une pluralité de graphes moléculaires associés à une pluralité de molécules et une pluralité d'étiquettes indicatives de propriétés chimiques de la pluralité des graphes moléculaires, dans lequel chaque graphe moléculaire de la pluralité de graphes moléculaires comprend une pluralité de noeuds cible, chaque noeud cible de la pluralité de noeuds cible étant connecté à une pluralité de noeuds sources pour faire passer des messages neuronaux à travers une pluralité d'arêtes, dans lequel les réseaux neuronaux d'attention de graphe conditionné par les arêtes permettent l'empilement de couches dans lesquelles chaque noeud dans le graphe moléculaire agrège des informations sur ses attributs de voisins de graphe local sur la base de la connectivité du graphe moléculaire et s'ajuste de manière adaptative à des noeuds de poids d'importance ;
le calcul de coefficients d'attention indicatifs d'une influence de la pluralité de noeuds sources sur un noeud cible de la pluralité de noeuds cible sur la base (1) d'une sortie softmax d'un produit matriciel d'un vecteur d'attention pouvant être appris et d'une matrice concaténée d'un état caché transformé linéairement du noeud cible, et (2) d'un produit matriciel d'informations d'arête transformées linéairement associées à une arête connectant le noeud source parmi la pluralité de noeuds sources au noeud cible, d'une différence relative de vecteurs d'état cachés du noeud source et du noeud cible par une matrice de poids pouvant être apprise, dans lequel les réseaux neuronaux d'attention de graphe conditionné par les arêtes effectuent une auto-attention sur les noeuds du graphe moléculaire de voisinage de taille différente et impliquent également un mécanisme d'attention partagé à travers les noeuds des graphes moléculaires pour calculer des coefficients d'attention, dans lequel les réseaux neuronaux d'attention de graphe conditionné par les arêtes sont agnostiques à la sélection de mécanisme d'attention et fonctionnent sur des entrées structurées de graphe de taille variable ;

le calcul d'une pluralité de messages neuronaux envoyés de la pluralité de noeuds sources au noeud cible en tant que produit d'une sommation d'un ensemble de vecteurs d'état cachés de la pluralité de noeuds sources et des informations d'arête transformées linéairement associées à l'arête connectant le noeud source parmi la pluralité de noeuds sources au noeud cible ;

la détermination d'une somme pondérée de la pluralité de messages neuronaux provenant de la pluralité de noeuds sources perçus par le noeud cible pour obtenir un vecteur de message unique agrégé à partir de la pluralité de noeuds sources, la somme pondérée étant déterminée par le biais des informations d'arête transformées linéairement en utilisant les coefficients d'attention ;

la transformation du vecteur d'état caché du noeud cible pour obtenir une intégration de niveau de noeud du graphe moléculaire, dans lequel la transformation du vecteur d'état caché du noeud cible comprend le calcul d'une sommation (1) du vecteur d'état caché transformé linéairement du noeud cible paramétré par le produit des coefficients d'attention et du paramètre de poids pouvant être appris, et (2) du vecteur de message unique agrégé à partir de la pluralité de noeuds sources ;

la détermination d'une intégration de niveau de graphe du graphe moléculaire en utilisant une fonction de lecture à partir de l'intégration de niveau de noeud du graphe moléculaire, dans lequel la fonction de lecture prend un graphe moléculaire transformé par attributs de noeud en tant qu'entrée et calcule une représentation de niveau de graphe ; et

le calcul des une ou plusieurs propriétés moléculaires en alimentant l'intégration de niveau de graphe du graphe moléculaire à une couche linéaire, dans lequel les réseaux neuronaux d'attention de graphe conditionné par les arêtes sont résilients au bruit par apprentissage pour s'adapter à l'élément pivot sur un fragment pertinent pour une tâche des graphes moléculaires à des champs réceptifs variables, une localité, une profondeur pour augmenter une puissance discriminante d'intégrations de noeud et de niveau de graphe, le noeud discriminant appris et les intégrations de niveau de graphe aident à la prédiction de propriétés moléculaires avec une complexité de calcul réduite.

12. Un ou plusieurs supports de stockage d'informations lisibles par machine non transitoires selon la revendication 11, comprenant en outre :

le fonctionnement sur le graphe moléculaire d'une phase de passage de message basée sur une agrégation de voisinage spatialement dynamique pour échanger la pluralité de messages neuronaux résultant en une transformation et une mise à jour de chaque intégration de niveau de noeud ; et

la réalisation d'une moyenne statistique de l'intégration de niveau de noeud correspondante obtenue à partir du réseau neuronal d'attention de graphe conditionné par les arêtes et de la phase de passage de message basée sur une agrégation de voisinage spatialement dynamique.

13. Un ou plusieurs supports de stockage d'informations lisibles par machine non transitoires selon la revendication 11, comprenant en outre :

le fonctionnement sur le graphe moléculaire d'une phase de passage de message basée sur des réseaux de convolution de graphe de mappage d'identité spatial pour échanger la pluralité de messages neuronaux résultant en une transformation et une mise à jour de chaque intégration de niveau de noeud ; et

la réalisation d'une moyenne statistique de l'intégration de niveau de noeud correspondante obtenue à partir du réseau neuronal d'attention de graphe conditionné par les arêtes et de la phase de passage de message basée sur des réseaux de convolution de graphe de mappage d'identité spatial.

14. Un ou plusieurs supports de stockage d'informations lisibles par machine non transitoires selon la revendication 11, comprenant en outre :

le fonctionnement sur le graphe moléculaire d'une phase de passage de message basée sur une propagation attentionnelle de graphe spatial pour échanger la pluralité de messages neuronaux résultant en une transformation et une mise à jour de chaque intégration de niveau de noeud ; et

la réalisation d'une moyenne statistique de l'intégration de niveau de noeud correspondante obtenue à partir du réseau neuronal d'attention de graphe conditionné par les arêtes et de la phase de passage de message basée sur une propagation attentionnelle de graphe spatial.

15. Un ou plusieurs supports de stockage d'informations lisibles par machine non transitoires selon la revendication 11, dans lequel les informations d'arête associées à l'arête du graphe moléculaire comprennent des caractéristiques de type d'arête de l'arête et une distance spatiale entre des atomes de graphe moléculaire qui sont représentés en tant que noeuds.

FIG. 1A

FIG. 1B

FIG. 1C

FIG. 1D

FIG. 2

ACCESS, VIA ONE OR MORE HARDWARE PROCESSORS, A
DATABASE COMPRISING A PLURALITY OF MOLECULAR GRAPHS
ASSOCIATED WITH A PLURALITY OF MOLECULES AND A
PLURALITY OF LABELS INDICATIVE OF CHEMICAL PROPERTIES OF
THE PLURALITY OF THE MOLECULAR GRAPHS, WHEREIN EACH
MOLECULAR GRAPH OF THE PLURALITY OF MOLECULAR GRAPHS
COMPRISES A PLURALITY OF SINK NODES, EACH SINK NODE OF
THE PLURALITY OF SINK NODES CONNECTED TO A PLURALITY OF
SOURCE NODES FOR PASSING NEURAL MESSAGES THROUGH A
PLURALITY OF EDGES

302

COMPUTE, VIA THE ONE OR MORE HARDWARE PROCESSORS,
ATTENTION COEFFICIENTS INDICATIVE OF INFLUENCE OF THE
PLURALITY OF SOURCE NODES ON A SINK NODE OF THE
PLURALITY OF SINK NODES BASED ON (1) A SOFTMAX OUTPUT OF
A MATRIX PRODUCT OF A LEARNABLE ATTENTION VECTOR AND
A CONCATENATED MATRIX OF A LINEARLY TRANSFORMED
HIDDEN STATE OF THE SINK NODE AND (2) A MATRIX PRODUCT
OF A LINEARLY TRANSFORMED EDGE-INFORMATION ASSOCIATED
WITH AN EDGE CONNECTING THE SOURCE NODE FROM AMONGST
THE PLURALITY OF SOURCE NODES WITH THE SINK NODE,
RELATIVE DIFFERENCE OF HIDDEN STATE VECTORS OF THE
SOURCE NODE AND THE SINK NODE BY A LEARNABLE WEIGHT
MATRIX

304

COMPUTE, VIA THE ONE OR MORE HARDWARE PROCESSORS, A
PLURALITY OF NEURAL MESSAGES SENT FROM THE PLURALITY
OF SOURCE NODES TO THE SINK NODE AS A PRODUCT OF THE
SUMMATION OF A SET OF HIDDEN STATE VECTORS OF THE
PLURALITY OF SOURCE NODE AND THE LINEARLY TRANSFORMED
EDGE-INFORMATION ASSOCIATED WITH THE EDGE CONNECTING
THE SOURCE NODE FROM AMONGST THE PLURALITY OF SOURCE
NODES WITH THE SINK NODE

306

A

300

FIG. 3A

A

DETERMINING, VIA THE ONE OR MORE HARDWARE PROCESSORS, A WEIGHTED SUM OF THE PLURALITY OF NEURAL MESSAGES FROM THE PLURALITY OF SOURCE NODES PERCEIVED BY THE SINK NODE TO OBTAIN AN AGGREGATED SINGLE-MESSAGE VECTOR FROM THE PLURALITY OF SOURCE NODES, THE WEIGHTED SUM DETERMINED THROUGH THE LINEARLY TRANSFORMED EDGE-INFORMATION USING THE ATTENTION COEFFICIENTS — 308

TRANSFORMING, VIA THE ONE OR MORE HARDWARE PROCESSORS, THE HIDDEN STATE VECTOR OF THE SINK NODE TO OBTAIN A GRAPH LEVEL EMBEDDING OF THE MOLECULAR GRAPH, WHEREIN TRANSFORMING THE HIDDEN STATE VECTOR OF THE SINK NODE COMPRISES COMPUTING A SUMMATION OF (1) THE LINEARLY TRANSFORMED HIDDEN STATE VECTOR OF THE SINK NODE PARAMETERIZED BY THE PRODUCT OF THE ATTENTION COEFFICIENTS AND THE LEARNABLE WEIGHT PARAMETER, AND (2) THE AGGREGATED SINGLE-MESSAGE VECTOR FROM THE PLURALITY OF SOURCE NODES — 310

DETERMINE, VIA THE ONE OR MORE HARDWARE PROCESSORS, A GRAPH LEVEL EMBEDDING OF THE MOLECULAR GRAPH USING A READ-OUT FUNCTION FROM THE NODE LEVEL EMBEDDING OF THE MOLECULAR GRAPH — 312

COMPUTE, VIA THE ONE OR MORE HARDWARE PROCESSORS, THE ONE OR MORE MOLECULAR PROPERTIES BY FEEDING THE GRAPH LEVEL EMBEDDING OF THE MOLECULAR GRAPH TO A LINEAR LAYER — 314

300

FIG. 3B

INPUT
DEVICES(S)
404

OUTPUT
DEVICES(S)
405

DEVICE(S)
409

I/O
INTERFACE
403

Tx/Rx 406

PROCESSOR
402

NETWORK
INTERFACE
407

COMMUNICATION
NETWORK 408

DEVICE(S)
410

STORAGE INTERFACE 412

RAM
413

RAM
414

MEMORY 415

APPLICATION DATA 418

USER INTERFACE 417

OPERATING SYSTEM 416

COMPUTER SYSTEM 401

401

FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202121046327

**Non-patent literature cited in the description**

- **JUSTIN GILMER et al.** Neural Message Passing for Quantum Chemistry. *COMPUTER SCIENCE*, 04 April 2017, 1-14
- Graph Attention Networks. **PETAR VELICKOVIC et al.** ARXIV.ORG. CORNELL UNIVERSITY LIBRARY, 30 October 2017
- Edge-Featured Graph Attention Network. **JUN CHEN et al.** ARXIV.ORG. CORNELL UNIVERSITY LIBRARY, 19 January 2021